# EUROPEAN PATENT SPECIFICATION

(11) **EP 1 680 174 B1**
(45) Date of publication and mention of the grant of the patent: **19.05.2010**
(21) Application number: 04775424.7
(22) Date of filing: 15.09.2004
(51) Int. Cl.: A61M 25/10

(54) **PARTIAL-LENGTH, INDWELLING PROSTATIC CATHETER WITH COILED INFLATION TUBE**
VERKÜRZTER PROSTATA-VERWEILKATHETER MIT SPULENFÖRMIGEM AUFBLASROHR
SONDE PROSTATIQUE A DEMEURE DE LONGUEUR PARTIELLE POURVUE D'UN TUBE DE GONFLAGE EN SPIRALE

(30) Priority: 17.09.2003 US 665742; 18.08.2004 US 921356
(43) Date of publication of application: 19.07.2006
(73) Proprietor: ProstaLund Operations AB, 226 60 Lund (SE)
(72) Inventor: SCHELIN, Sonny, S-380 30 ROCKNEBY (SE); BOLMSJÖ, Magnus, S-222 21 LUND (SE)
(74) Representative: Persson, Albin
(86) International application number: PCT/SE2004/001320
(87) International publication number: WO 2005/025665

(56) References cited:
- EP-A1- 0 733 379
- EP-A2- 0 935 977
- US-A- 3 983 879
- US-A- 5 716 373
- US-A1- 2002 173 741
- US-A1- 2002 173 818

## Description

This invention relates to a urinary catheter, and more particularly to a new and improved indwelling prostatic urethral catheter which uses a coiled section of an inflation tube as an anchor against movement of the catheter from its use position in the prostatic urethra, which has a urinary inlet configuration which reduces the incidence of obstructions in the urine flow path through the catheter, and which has a clearing device to facilitate removing and disintegrating obstructions, all of which improves the use and functionality of the catheter to drain urine from the bladder.

### Background of the Invention

Prostate problems, such as benign prostate hyperplasia (BPH) and malignant prostate cancer, are common occurrences among older men. The effects of these diseases are generally accompanied by swelling or enlargement of the prostate gland. Apart from the life-threatening aspects of malignant prostate cancer, the everyday symptoms and effects of these diseases are usually troublesome. One such problem relates to the ability to control and achieve normal urine discharge. When the prostate gland enlarges to the extent that it obstructs the prostatic urethra through the prostate gland, considerable difficulties arise in discharging urine at will. Such difficulties are typically referred to as urinary tract retention. Urinary tract retention can be either acute or chronic.

Surgical treatments are available for relieving urinary tract retention problems. Those treatments include thermotherapy and transurethral resection of the prostate (TURP). A TURP procedure involves surgically resecting tissue from the prostate gland to eliminate or reduce the obstruction or restriction. Surgical operations offer a high probability of an excellent clinical outcome, but they are associated with a high degree of morbidity. The alternative thermotherapy treatments are accompanied by milder side-effects. Thermotherapy treatments include transurethral microwave thermotherapy (TUMT), radio frequency needle ablation (TUNA), interstitial laser and hot water induced thermotherapy (WIT). All of the thermotherapy treatments involve heating the obstructive or restrictive prostatic tissue until the tissue is destroyed or damaged. Thereafter, the destroyed or damaged tissue sloughs off, or is absorbed in the body, resulting in an enlargement of the urinary passageway through the prostate gland. The enlargement of the urinary passageway through the prostate gland eliminates or relieves the obstruction and permits better urine flow.

Both thermotherapy and TURP surgical procedures cause temporary side effects, for example inflammation and swelling of the prostate. The side effects usually require the patient to use an indwelling urine drainage catheter for a few days up to several weeks following the procedure to permit urination while the swelling subsides and the tissue of the prostate gland heals or stabilizes. The tissue of the prostate gland which remains viable after the thermotherapy or TURP treatment is quite raw and tender, and direct contact from urine can aggravate the inflammation and increase the risk of infection. An indwelling catheter permits the urine to pass through the tissue where the surgical procedure was performed with only minimal or no contact with the treated tissue.

In those cases where the diseased prostate gland cannot be treated with thermotherapy or by a TURP procedure, the obstruction or restriction may become so significant that normal urinary functions are not possible or are only possible with great difficulty. In these circumstances, it is necessary for a catheter to be used for the rest of the patient's life. In some cases, the patient is taught to insert a full-length catheter whenever urination is necessary. In other cases where the patient cannot insert a full-length catheter himself, medical personnel must insert the full-length catheter in the urinary tract where it remains permanently until removed by medical personnel.

The typical type of urinary catheter used while the prostate gland heals, or on a continual basis, is a full-length urinary catheter. A full-length urinary catheter extends from the exterior opening of the urinary canal through the entire length of the urinary tract into the bladder. A clamp or other mechanical valve is attached at the exterior of the full-length catheter and is opened to drain the urine from the bladder. A reservoir may be attached to collect the urine, in which case the mechanical valve or clamp may not be used. The internal urinary sphincter muscle at the bladder neck, which normally controls the flow of urine from the bladder into the prostatic urethra and the urinary canal, is no longer able to perform its natural functions of constricting the urethra to stop urine flow and dilating to allow urine flow. TURP and heat treatment of surgical procedures usually destroy the internal urinary sphincter muscle. Even if not destroyed, neither the internal urinary sphincter muscle, which is located upstream of the prostatic urethra, nor the external urinary sphincter muscle, which is located downstream of the prostatic urethra, can control urine flow, because a continuously open urine flow passageway extends through the catheter. The urinary sphincter muscles cannot close the passageway through the full-length catheter.

In addition to the patient lacking the ability to naturally control urine flow when using a full-length urinary catheter, the extension of the catheter out of the exterior opening of the urinary canal and the presence of the clamp and the reservoir cause discomfort, are awkward to deal with and may be embarrassing for the patient. The full-length urinary catheter may create limitations from a social standpoint and almost always creates quality of life issues which must be confronted. Sexual activity is impossible. An increased risk of infection also results.

Because of the quality of life and social issues associated with full-length urinary catheters, partial-length indwelling catheters have been developed, see for example the catheter disclosed in EP-A-0 935 977. Partial-length indwelling catheters typically extend only from the bladder through the prostatic urethra, and not along the entire length of the urinary tract from the bladder to the exterior opening. The reduced length permits the external urinary sphincter muscle to control urine flow in a more natural manner, while still bypassing most of the urine flow around the swollen or raw prostate gland. No sizeable part of the catheter extends out of the exterior opening of the urinary canal.

Keeping a partial-length indwelling catheter in the proper position is essential. The short length may allow the catheter to move completely into the bladder or move out of the prostatic urethra into the urinary canal. Either type of unintended movement may require serious medical intervention to correct.

A partial-length urinary catheter typically uses an inflatable balloon or other form of anchor at its end which is innermost within the body and upstream relative to the urine flow. The balloon is expanded, or the anchor is enlarged, within the bladder, and the catheter is then is withdrawn until the balloon or anchor contacts the bladder neck at the entrance to the urethra. The balloon or anchor prevents the catheter from moving out of the bladder and into the urinary canal. However, the balloon cannot prevent the partial-length urinary catheter from moving out of the prostate gland and prostatic urethra and into the bladder.

One way of preventing a partial-length urinary catheter from moving into the bladder involves attaching a downstream anchor to the partial-length catheter with a short length of thread-like material. The thread-like material extends through the orifice of the external urinary sphincter muscle, and the downstream anchor is positioned downstream from the external urinary sphincter muscle. The catheter and the downstream anchor are positioned in the prostatic urethra and the urinary canal, respectively, on opposite sides of the external urinary sphincter muscle. The downstream anchor may be a short length of hollow tube or other three-dimensional structure which passes urine. The external urinary sphincter muscle is able to constrict around the thread-like material to stop urine flow and is able to dilate to permit the flow of urine, in a natural manner. The normal constricted state of the external urinary sphincter muscle adjacent to the downstream anchor prevents the partial-length catheter from moving into the bladder.

Inserting and removing the rigid tube or three-dimensional downstream anchor along with the partial-length urinary catheter may be difficult or painful. Special types of insertion tools and techniques are required to use partial-length urinary catheters with rigid tube and three-dimensional downstream anchors.

Because the partial-length prostatic urinary catheter must be inserted with the balloon deflated, all such balloon catheters must have some provision for inflating the balloon after the proper position of the catheter is attained. To inflate the balloon, a conduit or channel for adding fluid to the balloon must extend from the balloon to the exterior opening of the urinary tract. In partial-length of urinary catheters, the inflation tube is typically disconnected after the balloon is inflated and the inflation tube is thereafter entirely removed from the urinary canal. In a related context, the balloon must be deflated to remove the urinary catheter. Typically the balloon is deflated by opening a valve attached to the catheter. Opening the valve allows the fluid to escape from the balloon, so that the catheter can thereafter be removed. Should the valve not open when intended, medical intervention is required to deflate the balloon.

The inflated balloon may slowly lose the inflation fluid while the catheter is in use. Fluid loss may arise because the valve which confines the fluid to the balloon does not seal completely or because of slight pinhole breaches in the structural materials which form the balloon or seal it to the partial-length prostatic catheter. The risk of fluid loss and balloon deflation is increased with the increased time that the catheter remains in use. Any attempt to reinflate a balloon will generally require some form of medical intervention.

Another risk associated with the use of urinary catheters is an obstruction. A heat or surgical treatment of the prostate gland usually causes the destruction of the bladder neck and results in blood clots which enter the bladder. These clots float in the urine in the bladder and enter the catheter where they can accumulate to the extent of obstructing the flow of urine through the catheter. If the patient is unable to clear the clot accumulation by urinating, then the catheter must be medically removed and replaced. Replacement of the catheter shortly after a heat or surgical treatment causes irritation to the treatment area, can be painful for the patient, typically prolongs of the healing time period, and creates greater medical expense if the catheter must be removed and replaced. The problems of possible obstructions from blood clots are equally applicable to full-length and partial-length of catheters.

Obstructions may also arise from natural body substances in urine. While such risks are not as significant as those risks which arise because of blood clots after a surgical procedure, such risks are nevertheless significant for those individuals who must utilize a urinary catheter on a full-time basis.

These and other considerations, disadvantages and risks associated with the use of previous indwelling catheters have led to the improvements of the present invention.

### Summary of the Invention

In general, the present invention pertains to a full-length or partial-length urinary catheter which extends from the bladder through at least the prostatic urethra of the urinary tract to drain urine through the urinary canal of the urinary tract the exterior opening of the urinary canal. The catheter diverts a substantial majority of the urine flow out of contact with the prostate gland while still enabling the patient to control urine flow naturally with the urinary sphincter muscle. The retention of a partial-length catheter is facilitated, while minimizing discomfort and irritation to the urinary tract, by incorporating a coiled section of an inflation tube as a downstream anchor element. The inflation tube extends through the urinary canal to permit a balloon to be inflated in the bladder and function as an upstream anchor element. The coiled section of the inflation tube is resilient enough to develop holding force while the partial-length catheter is in use, but resiliently constricts to allow the catheter to be withdrawn from the urinary canal by pulling on the inflation tube. The partial-length catheter is inserted into position by a selectively disconnectable insertion tool which extends through the coiled section of the inflation tube.

The partial-length urinary catheter may be inserted immediately after a surgical or heat treatment procedure performed on the prostate gland, with diminished risks of obstructions and blood clots in the catheter, particularly after surgery. Configurations of one or more urine inlet openings into the catheter, and a selectively operable obstruction clearing device within the catheter, resist and eliminate the accumulation of blood clots and obstructions within the interior urine drainage passageway, thereby maintaining the urine drainage capability of the catheter. Fluid may also be flushed through the insertion tool and the catheter to reduce the risk of blood clot and other obstructions while the insertion tool remains connected to the catheter.

The present catheter is beneficial in avoiding entirely or reducing the number of instances where medical or surgical intervention is required to remove and replace an obstructed catheter. The present catheter also promotes better healing of the prostate gland after surgery because of the unobstructed passageway which conducts urine away from the surgically-affected area and thereby reduces the risk of infection and irritation. The improvements from the catheter also diminish the personal and social issues associated with the use of an indwelling catheter, by minimizing the size and amount of apparatus located at the exterior opening of the urinary tract.

These and other improvements and advantages are achieved by a catheter which is inserted in a urinary tract to drain urine from the bladder. The catheter of the present invention includes primary aspects and subsidiary aspects, which may be selectively used together in different forms of the invention.

The catheter drains urine to a location adjacent to an orifice in an external urinary sphincter muscle which controls urine flow into a urinary canal of the urinary tract. The catheter comprises a main body having a distal end, a proximal end and a length sufficient to position the distal end within the bladder and to position the proximal end adjacent to and distal of the orifice in the external urinary sphincter muscle. The main body defines a urine drainage interior passageway extending from the distal end to the proximal end. A distal anchor is attached to the distal end of the main body and is expandable within the bladder to restrain the main body against proximal movement within the urinary tract from a use position in which the distal end of the main body is located in the bladder and the proximal end of the main body is located adjacent to and distal of the orifice in the external urinary sphincter muscle. A proximal anchor is attached to the proximal end of the main body to restrain the main body against distal movement in the urinary tract from the use position. At least one urine inlet opening extends through the distal end of the main body and into the interior passageway. The cumulative cross-sectional sizes of all inlet openings in the distal end are greater than the cross-sectional sizes of the interior passageway to facilitate passing urine and blood clots from the bladder through the interior passageway and into the urinary canal.

The distal anchor comprises a balloon attached to the distal end of the main body of the catheter, and the balloon is expandable in size when inflated within the bladder to maintain the distal end of the main body in the bladder and to restrain the main body against proximal movement from the use position. An inflation tube is connected to the main body of the catheter. The inflation tube has a distal end, a proximal end and a length extending between the distal and proximal ends. The inflation tube extends from the main body through the orifice of the external urinary sphincter muscle and through the urinary canal to a position outside of an exterior opening of the urinary canal. The inflation tube and the main body establish an inflation passageway extending from the proximal end of the inflation tube to the balloon through which to deliver fluid to inflate the balloon. The proximal anchor comprises a coiled section of the inflation tube located at a position adjacent to and proximal of the sphincter muscle when the main body is in the use position. The coiled section interacts with the external urinary sphincter muscle when constricted to restrain the main body against distal movement from the use position.

An insertion tool may be connected to the main body to move the indwelling catheter within the urinary tract to the use position. The insertion tool has first and second opposite ends and a length sufficient to position the first end within the urinary tract distal of the sphincter muscle while the second end is at the exterior of the urinary canal. The coiled section of the inflation tube winds around the insertion tool when the insertion tool is connected to the indwelling catheter. A separable connection between the main body and the insertion tool connects the main body to the insertion tool to move the catheter and the insertion tool as a unit when positioning the indwelling catheter in the use position, but the separable connection permits separation of the main body from the insertion tool in response to continued proximal movement of the insertion tool when the expanded balloon restrains the main body against proximal movement from the use position. An internal channel extending between the first and second opposite ends of the insertion tool permits fluid communication with the interior passageway of the main body when the insertion tool is connected to the catheter at the separable connection to remove obstructions so long as the insertion tool remains connected to the catheter.

Various forms of various tip and inlet opening configurations into the interior passageway enhance the fluid draining capability of the catheter. One configuration of the inlet opening is defined by the absence of a longitudinal bisected half portion of a rounded tip to establish a whistle-tip shape. Another tip configuration is a plurality of longitudinally and circumferentially spaced inlet openings extending through the distal end of the main body. Two inlet openings may be positioned radially opposite one another on the distal portion of the main body, each of which has a cross-sectional size substantially greater than the cross-sectional size of the interior passageway, a circumferential dimension greater than one-fourth of the circumferential distance around the main body, and an elongated shape with the longer dimension extending longitudinally along the tip, thereby defining a couvelaire tip configuration. The distal end may include a beveled surface which extends substantially transversely at an acute angle relative to a longitudinal axis through the distal end, with a single inlet opening extending through the beveled surface substantially directly into the interior passageway of the main body.

A low friction coating may extend over the distal end of the main body, within each inlet opening, and along the interior passageway, to resist obstructions accumulating at each inlet opening and within the interior passageway.

The catheter may also include a clearing device having a contact element moveably positioned within the interior passageway of the main body of the catheter. The contact element is movable past an inlet opening of the catheter and along the interior passageway to contact and disintegrate and facilitate movement of blood clots and obstructions through the inlet opening and the interior passageway with the flow of urine.

An activation device is connected to the contact element and extends through the interior passageway and the urinary canal to a position outside of the exterior opening of the urinary canal. Manipulation of the activation device at the exterior opening of the urinary canal moves the contact element. The contact element may be removed from the interior passageway and out of the urinary canal by manipulation of the activation device. Alternatively a return element may be connected between the contact element and the main body to move the contact element back to an initial position out of the inlet opening after movement past the inlet opening and along the interior passageway. When used with an insertion tool having an interior channel, the activation device extends through the interior channel of the insertion tool when the insertion tool is connected to the main body.

The invention can be used in a method of draining urine from a bladder in a urinary tract which is preferably accomplished using features or functions exemplified by the catheter and its interaction with the urinary tract.

A more complete appreciation of the scope of the present invention as defined by claims 1, 10 and 20 and the manner in which it achieves the above-noted and other improvements can be obtained by reference to the following detailed description of presently preferred embodiments taken in connection with the accompanying drawings, which are briefly summarized below, and by reference to the appended claims.

### Brief Description of the Drawings

Fig. 1 is a perspective view of an indwelling prostatic catheter which incorporates the present invention, shown attached to an insertion tool and used with a syringe.
Fig. 2 is an enlarged perspective view of the indwelling prostatic catheter shown in Fig. 1 with the insertion tool removed and with a balloon of the indwelling catheter expanded.
Fig. 3 is an enlarged longitudinal cross-section view taken substantially in a longitudinal axial plane through Fig. 2.
Fig. 4 is an enlarged transverse cross-section view taken substantially in the plane of line 4-4 of Fig. 1.
Fig. 5 is an enlarged partial longitudinal axial cross-section view of a separable connection of the indwelling catheter-insertion tool assembly shown in Fig. 1, taken substantially in the plane of line 5-5 shown of Fig. 1.
Fig. 6 is a perspective view of the indwelling catheter and a portion of the insertion tool shown in Fig. 1, shown inserted within a urethra, a urethra sphincter, a prostatic urethra and a bladder of a urinary tract of a human being, with the physiology generally illustrated in cross section.
Fig. 7 is an illustration similar to Fig. 6 showing the balloon inflated within the bladder.
Fig. 8 is an illustration similar to Figs. 6 and 7 showing separation of the insertion tool from the indwelling catheter.
Fig. 9 is an illustration similar to Figs. 6-8 showing the position and use of the indwelling catheter.
Fig. 10 is a partial, enlarged and broken view of the catheter-insertion tool assembly shown in Fig. 1, illustrating an alternative embodiment of the separable connection shown in Fig. 5, and other aspects of the invention.
Fig. 11 is a perspective view of an indwelling catheter similar to that shown in Fig. 2 with a different distal end configuration.
Fig. 12 is a perspective view of an indwelling catheter similar to that shown in Fig. 11 with another distal end configuration.
Fig. 13 is a perspective view of an indwelling catheter similar to that shown in Figs. 11 and 12 with still another distal end configuration.
Fig. 14 is a perspective view of an indwelling catheter similar to that shown in Figs. 11-13 with a further distal end configuration.
Fig. 15 is a cross-section view of an indwelling catheter similar to that shown in Fig. 3 which further includes an obstruction clearing device located in a stowed position.
Fig. 16 is a cross-section view similar to that shown in Fig. 15, showing the clearing device moved longitudinally within an interior passageway of the catheter from the position shown in Fig. 15 to an operative position which clears obstructions.

### Detailed Description

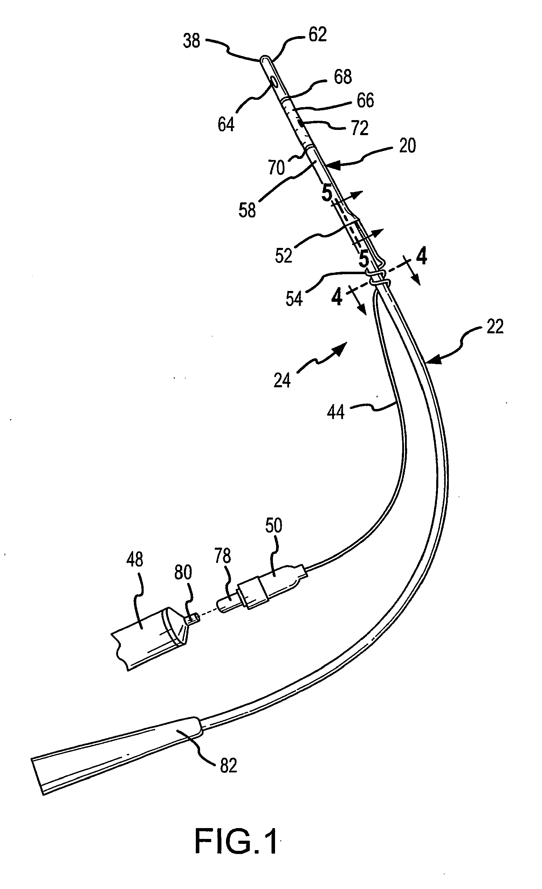

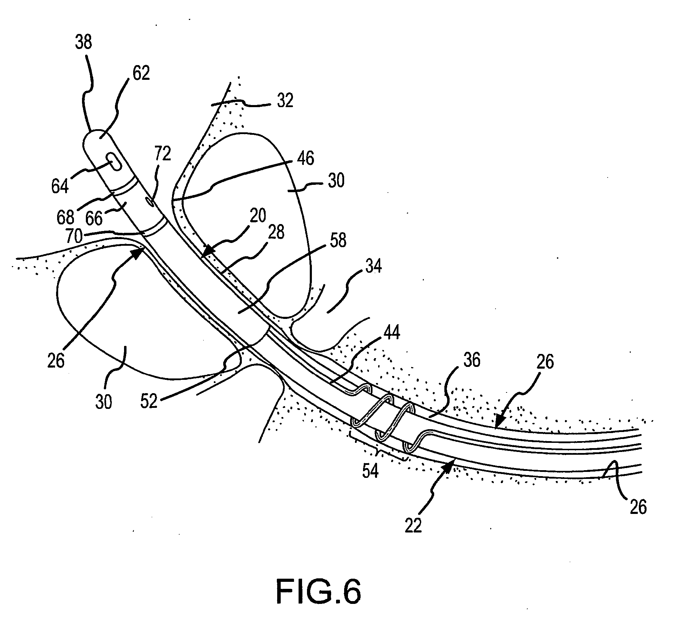

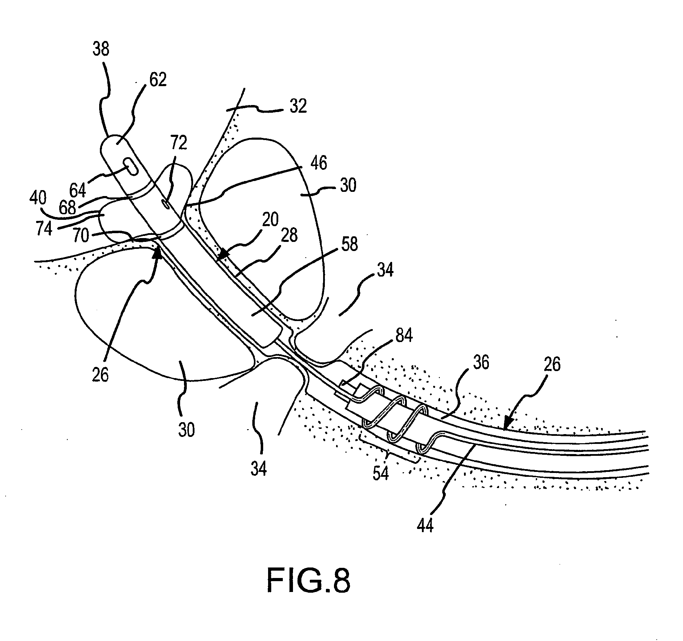

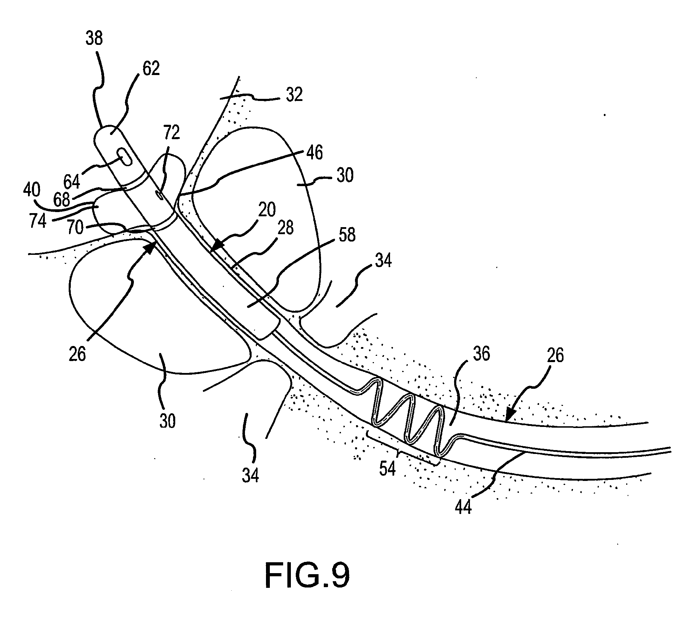

A partial-length indwelling catheter 20 which incorporates the present invention is shown in Fig. 1, but many aspects of the present invention are also applicable to a full-length catheters (not shown). The indwelling catheter 20 is connected to an insertion tool 22, to form a catheter-tool assembly 24, which allows the catheter 20 to be inserted into a urinary tract 26 of a human being, as shown in Fig. 6. Once inserted, the insertion tool 22 is disconnected or separated from the indwelling catheter 20 to leave the catheter 20 dwelling or remaining within a prostatic urethra 28 within a prostate gland 30, as shown in Fig. 9. In its indwelling use position shown in Figs. 8 and 9, the catheter 20 drains urine from a bladder 32 through the prostatic urethra 28 within the prostate gland 30 to a position slightly upstream of an external urinary sphincter muscle 34. When the external urinary sphincter muscle 34 dilates, the urine drains through an orifice of the external urinary sphincter muscle 34 and into a urinary canal 36 of the urinary tract 26.

The catheter 20 will typically be left in the indwelling position shown in Fig. 9 for a number of days or weeks to function as a temporary stent to protect raw tissue of the prostate gland 30, after it has been operated on to remove portions of the prostatic urethra 28 and the surrounding tissue of the prostate gland 30 in a transurethral resection of the prostate (TURP) surgical procedure or after these tissues have been destroyed or operated on by a microwave thermotherapy treatment (TUMT) or other thermotherapy treatment. In addition, the catheter 20 can also be used as a stent through the prostatic urethra 28 when it has been constricted to the point where urine will no longer pass effectively as a result of swelling in the prostate gland 30 due to benign prostate hyperplasia (BPH) or other obstructions or abnormalities. There are other medically-recognized reasons to use the indwelling catheter 20.

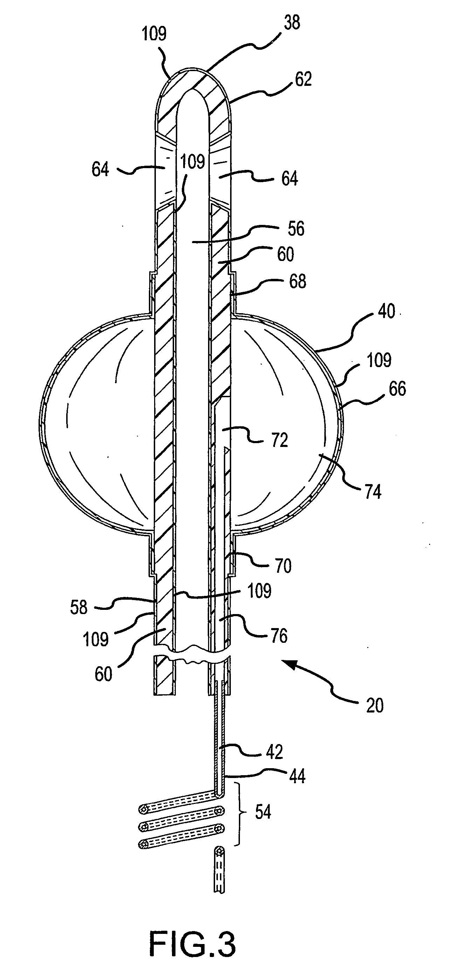

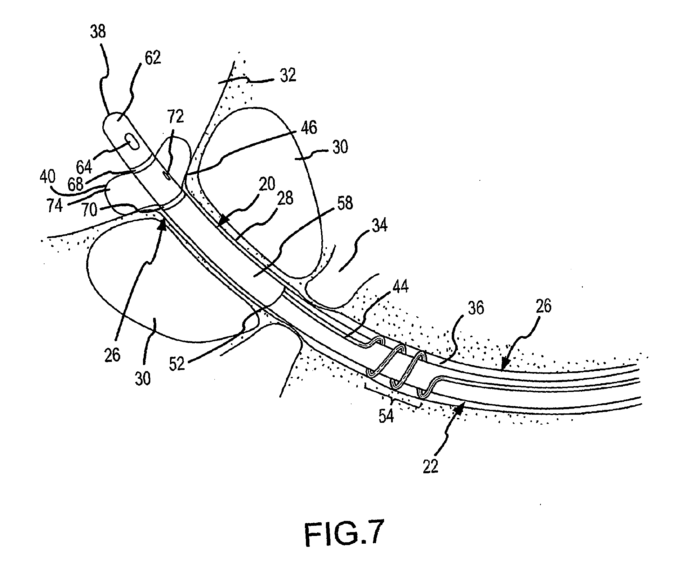

The indwelling catheter 20 is positioned as shown in Fig. 9 by pushing the catheter 20 up the urinary canal 36 into the urinary tract 26 with the insertion tool 22 until a distal end 38 of the indwelling catheter 20 enters the bladder 32 as shown in Fig. 6. A balloon 40 on the indwelling catheter 20 is inflated with fluid conducted through an inflation passageway 42 of an inflation tube 44 (Fig. 3) until the balloon 40 is larger in diameter than a neck 46 of the bladder 32 surrounding the prostatic urethra 28. The inflation fluid may be gas, such as air, or liquid such as saline solution. The balloon 40 is preferably inflated from an inflation pump such as a syringe 48 which is connected to a valve assembly 50 at the end of the inflation tube 44 (Fig. 1). Once the balloon 40 has been inflated, the insertion tool 22 is pulled backward until the inflated balloon 40 is seated on the bladder neck 46, as shown in Fig. 7. When seated on the bladder neck 46, the balloon 40 prevents the indwelling catheter 20 from moving out of the bladder 32, through the external urinary sphincter muscle 34 and into the urinary canal 36.

Continued withdrawal movement of the insertion tool 22 causes it to separate from the catheter 20 at a separable connection 52 (Fig. 1) between the indwelling catheter 20 and the insertion tool 22 as shown in Fig. 8, thereby leaving the indwelling catheter 20 in its final, indwelling position shown in Fig. 9. The insertion tool 22 is thereafter withdrawn and removed from within the urinary canal 36. The inflation tube 44 remains within the urinary canal 36.

The relative terms "proximal" and "distal" are used in this description in relation to the medical practitioner who inserts the catheter 20 into the urinary tract 26 at the exterior opening of the urinary canal 36. Accordingly, the portions of the catheter 20 which are the most internal within the patient are referred to as "distal," and the portions of the catheter 20 which are closest to the exterior opening of the urinary canal 36 are referred to as "proximal." The distal portions of the catheter are therefore located more interiorly within the urinary tract 26 than are the proximal portions of the catheter. The distal portions of the catheter are upstream relative to the normal direction of urine flow, and the proximal portions of the catheter are downstream relative to the normal direction of urine flow. The balloon 40 is located near the distal end of the catheter 20, and a distal end of the inflation tube 44 connects to a proximal end of a main body 58 of the catheter 20 at a location upstream or distal of the external urinary sphincter muscle 34.

The inflation tube 44 is formed with a permanently helically coiled section 54 shown in Figs. 1-3 and 6-9. The coiled section 54 is resilient both in the transverse dimension and in the longitudinal dimension. The inflation tube 44 has sufficient strength to maintain the coiled section 54 in the coiled configuration within the urinary canal 36 after removal of the insertion tool 22. Because of the resiliency of the coiled section 54, the coiled section 54 presses against the interior of the urinary canal 36. The orifice through the external sphincter 34 is normally closed except during urination thus preventing the coiled section 54 from migrating distally or upstream past the external sphincter 34. In this way, the coiled section 54 resists movement along the urinary canal 36, to hold the indwelling catheter 20 from moving distally along the urinary canal 36. By resiliently pressing against the interior of the urinary canal 36, the coiled section 54 also minimizes discomfort to the patient or irritation to the urinary canal 36. The coiled section 54 is not disruptive to the flow of urine through the urinary canal 36 because the coiled section 54 provides a fluid-flow path through an open center of the coils.

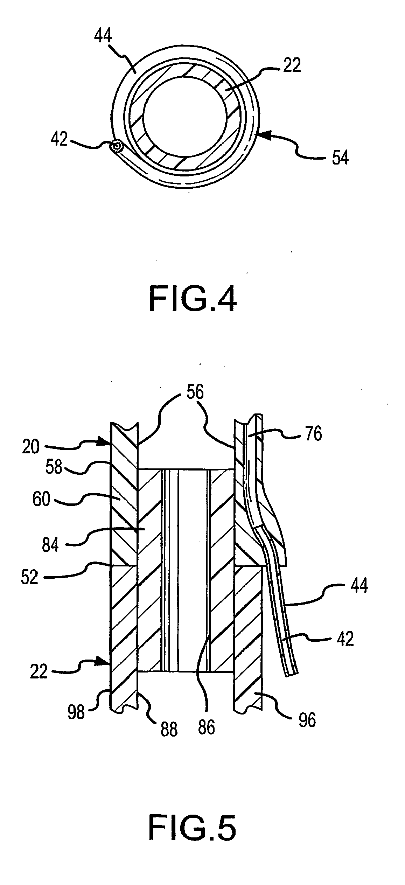

With the catheter 20 connected to the insertion tool 22, the coiled section 54 extends around the exterior of the insertion tool 22, as shown in Fig. 4. By extending around the exterior of the insertion tool 22, the coiled section 54 assists in holding the inflation tube 44 adjacent to the insertion tool 22 while the indwelling catheter 20 and the insertion tool 22 are inserted in the urinary tract 26. The coiled section 54 therefore assists in moving the inflation tube 44 into the urinary tract 26 along with the insertion tool 22. The helically coiled section 54 is loosely wound around the insertion tool 22, thereby allowing the insertion tool 22 to be withdrawn through the center of the coiled section 54 as the insertion tool 22 is disconnected from the indwelling catheter 20.

The coiled section 54 is located a short distance proximally from the proximal end of the main body of the indwelling catheter 20. The length of the inflation tube 44 between the proximal end of the indwelling catheter 20 and the coiled section 54 is sufficient to locate the coiled section 54 within the urinary canal 36 at a position slightly proximal of the urinary sphincter muscle 34, as shown in Figs. 6-9.

Located slightly proximally of the urinary sphincter muscle 34, the coiled section 54 of the inflation tube 44 functions as an anchor to assist in holding the indwelling catheter 20 in the urinary tract 26 in the position shown in Fig. 9. The coiled section 54 prevents the indwelling catheter 20 from moving distally from the position shown in Fig. 9, as a result of the coils of the coiled section 54 contacting a constriction in the urinary tract caused by constriction of the sphincter muscle 34. The coiled section 54 contacts the constriction to resist the distal movement of the indwelling catheter 20 and to prevent it from moving into the bladder 32. The inflated balloon 40 creates a restriction at the distal end 38 of the indwelling catheter 20 to prevent it from moving proximally along the urinary tract 26 and out of the urinary canal 36. With the inflated balloon 40 located at the distal end of the indwelling catheter 20 and the anchoring coiled section 54 located on the proximal side of the sphincter muscle 34, the indwelling catheter 20 resists movement either into or out of the bladder 32 and out of the prostatic urethra. Instead, the indwelling catheter 20 is maintained in the use position.

With the indwelling catheter 20 in the use position anchored by the balloon 40 and the coiled section 54, urine or other fluid from the bladder 32 is able to flow through an interior passageway 56 (Fig. 3) in the indwelling catheter 20 and out of the proximal end of the catheter 20 past the dilated urinary external sphincter muscle 34 (Fig. 9). The external urinary sphincter muscle 34 retains the capability to constrict or close around the inflation tube 44 to stop the urine flow. The urine is discharged into the urinary canal 36 in the normal manner. The final indwelling use position of the catheter 20 permits normal control of urine flow by the external urinary sphincter muscle 34.

More details concerning the structure of the indwelling catheter 20 are illustrated in Figs. 1-5. The indwelling catheter 20 includes a main or central body 58, preferably made from silicone rubber. The main body 58 has a generally cylindrical exterior shape. The main body 58 includes a sidewall 60 which defines the passageway 56 through the main body 58. An end piece 62 is either attached to or integral with the main body 58 at the distal end 38 of the catheter 20. The end piece 62 has a tip configuration adapted to facilitate insertion of the catheter 20 and the insertion tool 22 into the urinary tract 26. At least one and preferably a pair of urine inlet openings 64 are formed through the end piece 62. The openings 64 communicate between the exterior of the end piece 62 and the passageway 56 of the main body 58. Urine from the bladder 32 flows through the openings 64 and into and through the passageway 56 to the other proximal end of the main body 58.

The balloon 40 is formed by a flexible sleeve 66 of relatively thin, flexible, expandable, usually-transparent and non-porous material which is attached with fluid-tight seals 68 and 70 around the exterior of the main body 36. A first fluid-tight seal 68 is located slightly proximally of the distal end of the main body 58 where the end piece 62 is attached, and a second fluid-tight seal 70 is spaced proximally along the main body 58 from the first seal 68 by a distance approximately equal to the axial length of the flexible sleeve 66. The fluid-tight seals 68 and 70 are preferably formed by attaching the flexible sleeve 66 to the main body 58 with an adhesive or by thermal welding.

The flexible sleeve 66 is positioned over the top of and extends axially on opposite sides of an opening 72 from the main body 58. The fluid-tight seals 68 and 70 are located distally and proximally of the opening 72, respectively. Fluid is introduced into a volume 74 at the exterior of the main body 58 between the fluid-tight seals 68 and 70 and within the flexible sleeve 66, causing the flexible sleeve 66 to expand outward and create the balloon 40.

An inflation conduit 76 communicates with the opening 72, as shown in Fig. 3. The inflation conduit 76 is formed within the sidewall 60 of the main body 58. A distal end of the inflation tube 44 is inserted into a proximal end of the inflation conduit 76, as shown in Figs. 3 and 5. The fluid is delivered from the syringe 48 (Fig. 1) into the inflation passageway 42 of the inflation tube 44 and flows into the inflation conduit 76, out of the opening 72 and into the volume 74 beneath the flexible sleeve 66, causing the flexible sleeve 66 to expand into the form of the balloon 40.

Inserting the distal end of the inflation tube 44 into the inflation conduit 76, as shown in Fig. 5, allows the inflation tube 44 to bypass or go around the separable connection 52 between the indwelling catheter 20 and the insertion tool 22. A strong fluid-tight bond is formed by attaching the inflation tube 44 into the inflation conduit 76 with an adhesive. The attachment maintains the inflation tube 44 connected to the main body so that pulling on the inflation tube from the exterior of the urinary canal 36 will remove the catheter 20 from the urinary tract 26 without the inflation tube 44 breaking away from the main body 58. In this regard the inflation tube 44 also serves as a tether for the catheter 20. The separable connection 52 shown in Fig. 5 permits the communication between the inflation passageway 42 and the inflation conduit 76 to remain intact and fluid tight after the insertion tool 22 has separated from the indwelling catheter 20 while the catheter 20 remains positioned within the urinary tract. The continued integrity of the inflation passageway between the balloon 40 and the valve assembly 50 allows the balloon 40 to be periodically reinflated while the indwelling catheter 20 is in use, if necessary. Periodic reinflation may be necessary as a result of minute leaks in the balloon 40, the valve assembly 50 or the passageways connecting the balloon 40 and the valve assembly 50.

The inflation tube 44 has a length which extends from the main body 58 of the indwelling catheter 20 through the urinary canal 36 to the outside of the human body. The length of the inflation tube 44 is sufficient to position the valve assembly 50 at the exterior of the human body. The inflation tube 44 has sufficient rigidity to prevent the inflation passageway 42 within the tube 44 from collapsing from contact with the tissue of the urinary tract 26, but the rigidity is not so great as to prevent a moderate amount of flexibility in the inflation tube 44. The moderate flexibility of the inflation tube 44 allows it to extend through the typical curves of the urinary tract 26.

The valve assembly 50 is of conventional construction and includes a receptacle 78 into which a nozzle 80 of the syringe 48 is inserted, as shown in Fig. 1. The valve assembly 50 also includes a conventional internal check valve (not shown) which closes the inflation passageway 42 at the valve assembly 50 when the nozzle 80 is removed from the receptacle 78. In this manner, fluid from within the balloon 40 is prevented from escaping through the inflation passageway 42 when the syringe 48 is disconnected from the valve assembly 50, but the check valve permits fluid from the syringe 48 to inflate the balloon 40 when a plunger (not specifically shown) of the syringe 48 is depressed. Thus, the balloon 40 will remain inflated after the syringe 48 is disconnected from the valve assembly 50. However, should the balloon 40 need to be reinflated or should additional fluid need to be added to expand the balloon 40 during use of the catheter, the syringe 48 is easily connected to the valve assembly 50 for doing so.

As an alternative to the use of the valve assembly 50, the inflation passageway 42 can be sealed at a proximal end after the balloon 40 has been inflated. For example, instead of using the valve assembly 50 to prevent fluid from escaping from the balloon, a knot may be tied or an obstruction formed (neither shown) in the proximal end of the inflation tube 44 at a location spaced proximally from the end of the penis at the external opening of the urinary canal. The knot or obstruction seals the inflation passageway 42 and prevents the fluid from escaping through the passageway to maintain the balloon 40 inflated. The inflation tube 42 is cut at a position slightly proximally of the knot or obstruction. In this alternative configuration, the inflation tube 44 without the valve assembly 50 extends only a modest distance from the open end of the urinary canal 36. Greater comfort and convenience is promoted because there is no sizable apparatus to deal with, such as the valve assembly 50 connected to the proximal end of the inflation tube 44. If the balloon 40 needs to be reinflated or have additional fluid added after the indwelling catheter 20 has been used for some time, the knot or obstruction can be cut from the end of the inflation tube 44 and a suitable connector attached to allow the syringe 48 to introduce additional fluid. After suitable inflation, another knot can be tied or an obstruction formed in the remaining proximal portion of the inflation tube 40. Releasing the fluid through the inflation passageway 42 collapses the balloon 40 and allows the catheter 20 to be pulled out of the urinary canal 36 by pulling on the inflation tube 44.

The insertion tool 22 is a flexible tubular structure and is generally configured similar to the distal portion of a typical full-length urinary catheter. The insertion tool 22 is at least long enough to extend from outside of the body into the urinary canal 36 and prostatic urethra 28 to a point that will place the indwelling catheter 20 in the final desired use position. The insertion tool 22 is preferably made from silicone rubber, but has sufficient structural integrity to transfer pushing forces supplied from the outside of the body longitudinally along the length of the insertion tool 22, thereby allowing the insertion tool 22 with the attached indwelling catheter 20 to be moved distally into the urinary tract 26. A proximal end of the insertion tool 22 may take the form of a hollow handle 82 or enlargement, by which to grip the insertion tool 22 and apply pushing force to it during insertion in the urinary tract 26.

The separable connection 52 between the insertion tool 22 and the indwelling catheter 20 includes a sleeve 84, shown in Fig. 5. The sleeve 84 is rigidly connected to the distal end of the insertion tool 22 by an adhesive, for example. A distal portion of the sleeve 84 projects beyond the distal end of the insertion tool 22 and into the interior passageway 56 of the catheter. The distal portion of the sleeve 84 has an exterior diameter which frictionally fits within the interior passageway 56, and the friction created by the insertion of the sleeve 84 into the interior passageway 56 is sufficient to retain the indwelling catheter 20 to the insertion tool 22 during manipulation of the catheter-tool assembly 24 within the urinary tract 26 during insertion and placement, prior to inflation of the balloon 40. However, the degree of frictional resistance between the distal end of the sleeve 84 and the main body 58 at the proximal end of the interior passageway 56 is not so great as to prevent the indwelling catheter 20 from separating from the insertion tool 22 once the balloon 40 has been inflated and seated against the bladder neck 46. The amount of frictional resistance between the distal portion of the sleeve 84 and the proximal end of the main body 58 at the proximal end of the interior passageway 56 can be increased by forming serrations on the exterior of the distal end of the sleeve 84. The resilient material of the main body 58 will deform slightly around the serrations to further assist in holding the indwelling catheter 20 to the insertion tool 22, but the amount of deformation is not so great as to prevent separation of the indwelling catheter 20 and the insertion tool 22 at the separable connection 52. The sleeve 84 has a center opening 86 formed through it to provide a fluid flow path from the passageway 56 through the sleeve 84.

The catheter-tool assembly 24 is inserted and used in the manner illustrated in Figs. 6-9. As shown in Fig. 6, the catheter 20 and the insertion tool 22 are inserted into the urinary tract 26 through the urinary canal 36, in a manner similar to the way that a conventional full-length urinary catheter would be inserted. The insertion force is applied by pushing on the insertion tool 22 and on the handle 82 attached at its proximal end. Distal movement of the catheter-tool assembly 24 continues until the rounded end piece 62 and a significant distal portion of the indwelling catheter 20 are located in the bladder 32. The insertion is sufficient to assure that the flexible sleeve 66 will be located within the bladder 32. To assure sufficient insertion, it is frequently the case that the distal movement continues until terminated when the end 38 contacts the opposite wall of the bladder 32, thereby assuring that the balloon 40 is within the bladder 32. During insertion in this manner, the coiled section 54, which is wrapped around the insertion tool 22, helps keep the forward or distal portion of the inflation tube 44 aligned with and progressing with the indwelling catheter 20.

Once the catheter-tool assembly 24 has been inserted sufficiently, the balloon 40 is inflated as shown in Fig. 7. Inflation is achieved by connecting the syringe 48 to the valve assembly 50, and depressing the plunger (not shown) of the syringe 48 to force fluid through the inflation passageway 42 of the inflation tube 44, into the inflation conduit 76, through the opening 72 and into the interior volume 74, causing the flexible sleeve 66 to expand into the balloon 40. After the balloon 40 is in the expanded position, the insertion tool 22 is pulled to move the catheter-tool assembly 24 in the proximal direction until the inflated balloon 40 seats against the bladder neck 46.

With the balloon 40 seated against the bladder neck 46, continued proximal movement of the insertion tool 22 causes the separable connection 52 to separate the indwelling catheter 20 from the insertion tool 22, as shown in Fig. 8. The balloon 40 prevents the indwelling catheter 20 from coming out of the urinary tract 26 with the insertion tool 22 because the expanded balloon 40 is larger than the bladder neck 46. The coiled section 54 of the inflation tube 44, being located proximally from the external urinary sphincter muscle 34, prevents the indwelling catheter 20 from moving into the bladder 32. The continued withdrawal of the insertion tool 22 is not inhibited by the coiled section 54, because the body of the insertion tool 22 moves through the interior of the coiled section 54. The length of the inflation tube 44 is sufficient to locate the valve assembly 50 at the exterior of the urinary tract 26.

After the insertion tool 22 is removed as shown in Fig. 9, the balloon 40 remains inflated in the bladder 32, and the proximal end of the main body 58 of the indwelling catheter 20 extends through the prostatic urethra 28 but does not extend through the external urinary sphincter muscle 34. The coiled section 54 is located on the opposite or proximal side of the external urinary sphincter muscle 34. In this final position, the balloon 40 prevents the indwelling catheter 20 from moving out of the prostatic urethra 28 and into the urinary canal 36, while the coiled section 54 prevents the indwelling catheter 20 from moving out of the prostatic urethra 28 and into the bladder 32. The inflation tube 44 does not interfere with the ability of the urinary sphincter muscle 34 to constrict around the inflation tube 44 and naturally stop the urine flow. Similarly, the inflation tube 44 does not interfere with the natural ability of the external urinary sphincter muscle 34 to dilate, as shown in Fig. 9, and thereby naturally allow urine to flow through the interior passageway 56 of the indwelling catheter 20 and into the urinary canal 36. In this manner, the inflation tube 44 does not interfere with the natural control functions of the external urinary sphincter muscle 34.

When the urinary sphincter muscle 34 is dilated, the urine flows from the bladder 32 through the openings 64 and into the interior passageway 56 in the main body 58, as understood from Fig. 3. The flow of urine through the interior passageway 56 largely bypasses the prostatic urethra 28 or the prostate gland tissue if the prostatic urethra 28 has been removed by a surgical procedure. The dilated urinary sphincter muscle 34 (Fig. 9) allows urine to flow from the interior passageway 56 of the indwelling catheter 20 through the urinary canal 36 in the normal manner. Constriction of the urinary sphincter muscle 34 around the inflation tube 44 stops the urine flow.

While the urinary sphincter muscle 34 is dilated to permit urine flow, the balloon 40 prevents the indwelling catheter 20 from exiting the bladder 32 and moving into the dilated urinary sphincter muscle 34. The contact of the coiled section 54 against the urethra of the urinary canal 36 resists movement of the indwelling catheter 20 into and out of the bladder 32. In addition, the inflation tube 44 can be held at the valve assembly 50 to resist distal movement of the indwelling catheter 20 during urination when the urinary sphincter muscle 34 is dilated, if necessary or desirable. In this manner, the indwelling catheter 20 stays in position even when the urinary sphincter muscle 34 is dilated.

When the indwelling catheter 20 is no longer needed, typically as a result of natural healing of the prostate gland 30 after a surgical or heat treatment procedure, or if it is necessary to periodically replace the indwelling catheter 20, removal is accomplished after deflating the balloon 40. Deflation is accomplished by inserting the syringe 48 into the valve assembly 50 and moving the plunger (not shown) of the syringe 48 outward to withdraw fluid from the inflation passageway 42. The insertion of the syringe 48 in the valve assembly 50 opens the check valve within the valve assembly 50 and allows the fluid to be withdrawn. If the inflation tube 44 has been tied into a knot or an obstruction has been formed to avoid use of the valve assembly 50 in the manner described above, the inflation tube 44 may be cut at a location distal of the knot or obstruction to allow the fluid to escape. The escaping fluid causes the balloon 40 to deflate, and the flexible sleeve 66 moves to a collapsed position (shown in Figs. 1 and 6) adjacent to the main body 58 of the indwelling catheter 20.

Once the balloon 40 has been deflated, the inflation tube 44 is pulled outward by gripping and pulling on the valve assembly 50 or the proximal end of the inflation tube 44. Force is transferred through the inflation tube 44 to the main body 58 of the indwelling catheter 20. The pulling force constricts and elongates the coils of the coiled section 54, thereby reducing their transverse dimension as a result of longitudinally separating the individual coils with the pulling force. The reduced transverse dimension lessens or eliminates contact with the urinary canal 36. In this manner the coiled section does not inhibit removal of the catheter or induce significant discomfort as it moves through the urinary canal. The amount of force transferred is sufficient to move the main body 58 of the indwelling catheter 20 past the urinary sphincter muscle 34 and into the urinary canal 36. The deflated balloon 40 does not resist movement of the distal end of the indwelling catheter 20 through the bladder neck 46. Continued pulling movement on the inflation tube 44 moves the indwelling catheter 20 through the urinary canal 36 until the indwelling catheter 20 is completely withdrawn from the proximal end of the urinary canal 36.

After many TURP and heat treatment procedures, blood from the affected tissue tends to accumulate in the bladder. The blood flows out of the bladder during natural urination. With the indwelling catheter 20 located in the use position described above, blood could clot within the interior passageway 56 of the indwelling catheter 20 or blood clots might accumulate to the degree that the interior passageway 56 or openings 64 are blocked or obstructed. Natural pressure from the bladder may be insufficient to overcome the blockage, thereby preventing urine flow from the bladder 32 through the catheter 20. Under such circumstances, it is necessary to remove the obstructed catheter and insert a new catheter, in the manner described above. Replacing an obstructed catheter is to be avoided if possible, because the removal and insertion further aggravates the already-tender tissue within the prostate gland that has been affected by the surgical procedure.

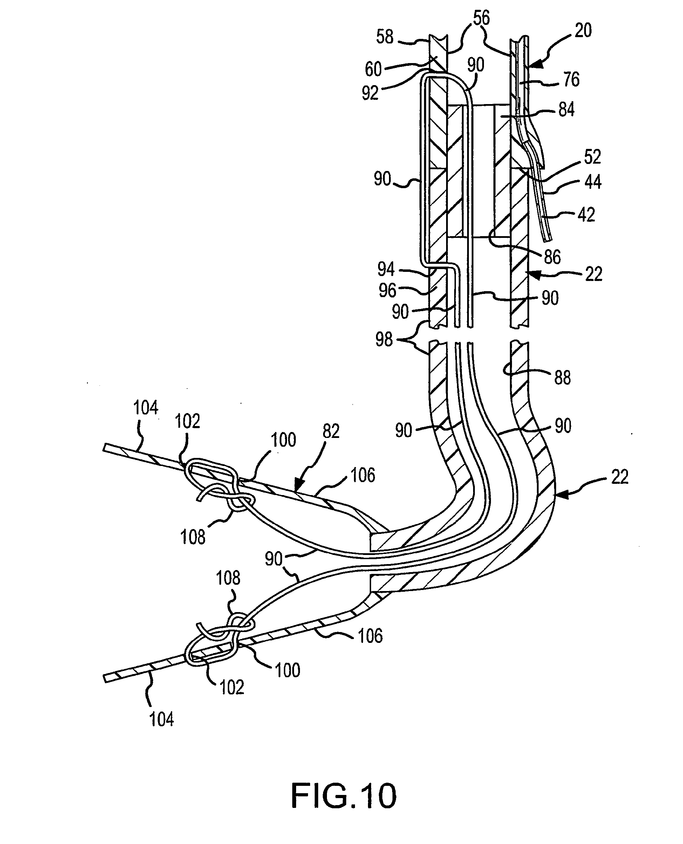

One approach which avoids replacing the catheter under circumstances where blood clots accumulate in the interior passageway 56 is to flush the interior passageway 56 with saline or other appropriate liquid on a periodic basis. To do so, it is necessary to establish fluid communication through the urinary canal 36, the constricted external urinary sphincter muscle 34 and into the interior passageway 56 of the catheter 20. The most direct manner of establishing this type of fluid communication is to maintain the insertion tool 22 connected to the indwelling catheter 20, so that the flushing fluid can be supplied through an interior channel 88 of the insertion tool 22 from the handle 82, as understood from Fig. 10. The flushing fluid passes through the interior channel 88 and through the center opening 86 of the sleeve 84 and into the passageway 56 of the indwelling catheter 20. The flushing fluid forces any blood clots which accumulated in the interior passageway 56 back into the bladder 32 (Fig. 6) or dissolves the clots within the interior passageway 56. Removing the clots relieves the obstruction and allows urine to flow through the indwelling catheter in the manner described.

Until the affected tissue stops bleeding, there is a risk that the clots will continue to form within the passageway 56. The risk of obstruction from blood clots is normally the greatest within the first twenty-four hours after a TURP or heat treatment procedure. During this time period, it is desirable to provide the ability to flush the interior passageway 56. To provide the ability to flush the interior passageway, the insertion tool 22 must remain connected to the indwelling catheter 20 during this time so that the interior channel 88 of the insertion tool 22 remains in fluid communication with the interior passageway 56, through the center opening 86 of the sleeve 84.

Instead of pulling the insertion tool 22 in the proximal direction to separate the insertion tool 22 from the indwelling catheter 20 at the separable connection 52 after the indwelling catheter has been located in the use position (Fig. 8), the insertion tool 22 may be left in place for approximately the first twenty-four hours. The natural friction between the proximal end of the main body 58 of the indwelling catheter 20 and the distal portion of the sleeve 84 connected to the insertion tool 22 may be sufficient to maintain the insertion tool 22 connected to the indwelling catheter in some circumstances. However in other circumstances, the patient may be released from the supervision of medical personnel immediately after the procedure and will be ambulatory during the first twenty-four hour period after the procedure. Movement of the patient under these circumstances may tend to create enough force on the insertion tool 22 to disconnect the insertion tool from the indwelling catheter prematurely before the risk of blood clots has subsided.

To assure that the insertion tool 22 remains connected to the indwelling catheter 20 at the separable connection 52 until medical personnel determines that it is appropriate to separate the indwelling catheter 20 from the insertion tool 22, a bridging structure, preferably in the form of a relatively fine thread-like cord 90, is used to maintain temporarily the separable connection 52 of the indwelling catheter 20 and the insertion tool 22, as shown in Fig. 10. The cord 90 extends from the interior passageway 56 through a small hole 92 in the sidewall 60 of the main body 58 of the indwelling catheter 20. From the hole. 92, the cord 90 extends across and bridges the separable connection 52 along an outside surface of the main body 58 of the indwelling catheter and along an outside surface of a tubular main body 98 of the insertion tool 22. The cord also extends through another small hole 94 in a sidewall 96 of a main tubular body 98 of the insertion tool 22, and into the interior channel 88. The two opposite free-end portions of the cord 90 extend along the length of the interior channel 88 and terminate within the hollow interior of the handle 82. The terminal end portions of the cord 90 each loop through small holes 100 and 102 formed in a sidewall 104 of a main body 106 of the handle 82. Knots 108 are formed in the terminal end portions of the cord 90 within the hollow interior of the handle 82 to hold each end of the cord 90 in place relative to the handle 82.

Any slack longitudinal length of the cord 90 is eliminated when the knots 108 are tied in the terminal ends of the cord 90. Eliminating any longitudinal slack in the cord 90 pulls together the proximal end of the indwelling catheter 20 and the distal end of the insertion tool 22. The cord 90 therefore prevents the indwelling catheter 20 from separating from the insertion tool 22 at the separable connection 52. The communication channel through the interior channel 88, the center opening 86 of the sleeve 84 and the interior passageway 56 is maintained so long as the cord 90 connects the indwelling catheter 20 and the insertion tool 22.

While the indwelling catheter 20 is maintained connected to the insertion tool 22, flushing fluid may be forced from the handle 82 through the interior channel 88, through the center opening 86 and into the interior passageway 56. The flushing fluid dissolves any clots within the interior passageway 56, and keeps the passageway 56 unobstructed for urine flow from the bladder. The dissolved clots flow from the interior passageway 56 through the interior channel 88, or the clots may be pushed back into the bladder with the flushing fluid. Of course, while the open communication path exists through the insertion tool 22 and the indwelling catheter 20, urine from the bladder will also flow through this open path. To control the urine flow under these circumstances, it is necessary to insert a valve in the interior channel 88 at the handle 82, to control the discharge of the urine. The extension of the insertion tool 22 through the sphincter muscle 34 prevents the sphincter muscle from naturally controlling the urine flow.

When it is determined that the risk of blood clots has diminished and it is no longer necessary or desirable to flush the interior passageway 56 of the indwelling catheter 20, preferably at least twenty-four hours after the procedure, one or both of the knots 108 is untied or cut. One end of the cord 90 is pulled in the proximal direction, which moves the other free end of the cord into the interior channel 88, through the holes 92 and 94 and then out of the interior channel 88, thereby disconnecting the indwelling catheter 20 from the insertion tool 22 at the separable connection 50. Once the cord 90 no longer holds the indwelling catheter and the insertion tool together as shown in Fig. 10, the insertion tool 22 is pulled in the proximal direction to separate it from the indwelling catheter 20 in the manner previously described and shown in Fig. 8. The indwelling catheter 20 remains in the use position shown in Fig. 9.

The resistance to clotting, both before and after the insertion tool 22 is removed from the indwelling catheter 20, is enhanced by coating the indwelling catheter 20 and portions of the insertion tool 22 with a layer or coating 109 of low friction material, such as parylene or a hydrophilic material, as shown in Fig. 3. The coating 109 of the low friction material is applied to the main body 58, to cover its sidewall 60 on the exterior and interior, including the passageway 56, the end piece 62 and the openings 64 within the end piece 62. In other circumstances, an outer layer 109 of the low friction material may be integrally made a part of the sidewall 60, including the passageway 56 and the openings 64. The low friction material reduces the adherence of the blood clots within the openings 64 and in the passageway 56, and reduces the frictional force holding those blood clots in the openings 64 and the passageway 56. The reduced adherence or frictional holding force makes it easier to dislodge the clots, with natural fluid pressure from the bladder or when flushing the indwelling catheter with fluid through the insertion tool in the manner previously described, or in conjunction with the action of a clearing device described below. The low friction material on the interior surface of the passageway 56 also reduces the resistance to the flow of urine and clots or other potential obstruction through the passageway 56. The low friction material on the exterior surface of the main body 58 and on the exterior surface of the insertion tool 22 facilitates insertion of the indwelling catheter in the urinary canal 36.

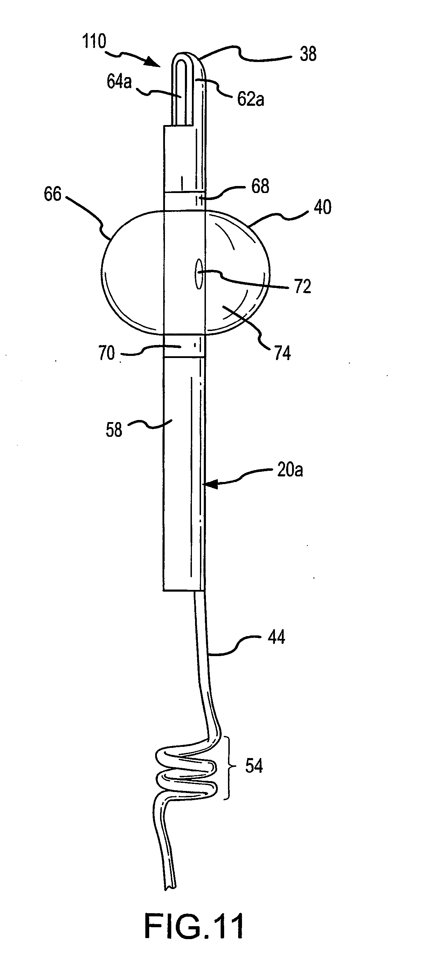

Another technique for obtaining increased resistance to blood clot obstructions of the interior passageway 56 within the catheter 20 is to increase the size of the inlet opening or openings 64 through the end piece 62, or to orient the inlet opening 64 in a manner which inhibits clot accumulation, or to do both. Preferably, the cross-sectional size of the inlet opening or the cumulative cross-sectional sizes of all of the inlet openings should be substantially greater than the cross-sectional size of the interior passageway 56, preferably at least two to four or more times greater. Indwelling catheters 20a-20d which employ inlet openings 64 of increased size and direct orientation are shown in Figs. 11-14. The layer or coating 109 (Fig. 3) of the low friction material may also be used in conjunction with the catheters 20a-20d shown in Figs. 11-14.

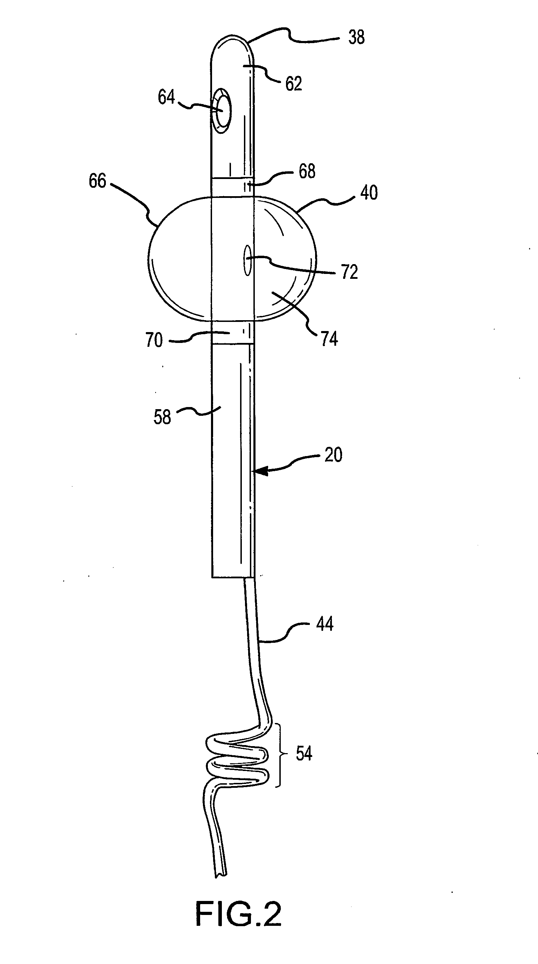

The catheter 20a shown in Fig. 11 has a single enlarged urine drainage inlet opening 64a formed in the end piece 62a. The end piece 62a retains the longitudinal half structure or portion of the rounded distal end 38 of the end piece 62 (Figs. 2 and 3), but the other longitudinal half portion is eliminated for a substantial longitudinal distance extending proximally from the most distal point of the rounded end 38 to create the substantially enlarged inlet opening 64a. The shape of the end piece 62a and the inlet opening 64a cause the distal end of the indwelling catheter 20a to assume the shape or configuration of a whistle. For that reason, the catheter 20a is referred to as having a whistle tip 110. The substantially enlarged inlet opening 64a into the interior passageway 56 (Fig. 3) from the bladder 32 makes it difficult for blood clots to accumulate within the inlet opening 64a and to obstruct urine flow into and through the passageway 56. The rounded distal end portion of the whistle tip 110 facilitates the insertion of the catheter 20a into the urinary canal 36.

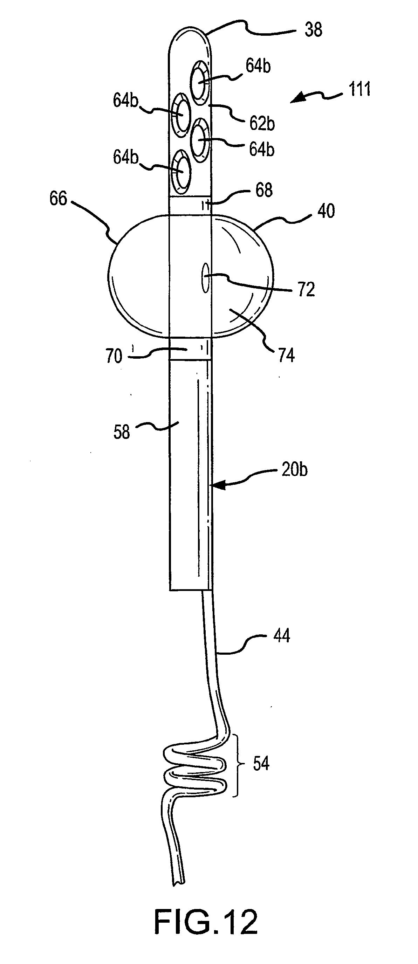

The indwelling catheter 20b shown in Fig. 12 has multiple urine drainage inlet openings 64b spaced at longitudinal and circumferential positions around the end piece 62b. Each of the inlet openings 64b communicates from the exterior of the end piece 62b into the interior passageway 56. Each of the inlet openings 64b is approximately the same size as, or slightly smaller than, each of the pair of inlet openings 64 in the rounded distal end 38 of the end piece 62 (Figs. 2 and 3), but the multiplicity of the inlet opening 64b provides a cumulatively larger pathway for the urine to pass from the bladder into the passageway 56 of the catheter 20b. Having a multiplicity of inlet openings 64b rather than a single inlet opening 64 or a pair of inlet openings 64 decreases the possibility that a blood clot will prevent urine from flowing into the interior passageway 56 (Fig. 3) from the bladder 32, and decreases the possibility of a single clot entering the catheter 20b and blocking the passageway 56 (Fig. 3). In the event that one or more of the inlet openings 64b becomes obstructed with a blood clot, urine will flow into the passageway 56 through one or more of the other unobstructed openings 64b. The multiple urine drainage inlet openings 64b in the catheter 20b cause it to be referred to as having a multiple opening tip 111. The rounded distal end 38 of the multiple opening tip 111 facilitates the insertion of the catheter 20b into the urinary canal 36.

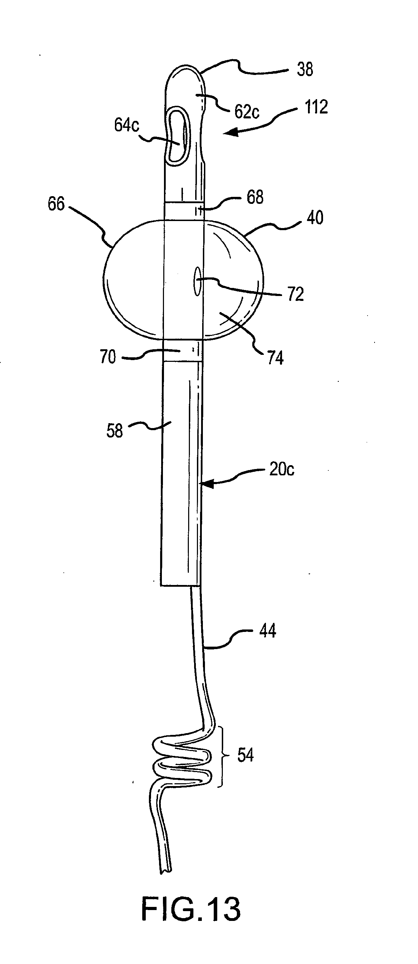

The indwelling catheter 20c shown in Fig. 13 has a couvelaire tip 112. The couvelaire tip 112 has a pair of relatively-large elongated or oval-shaped, inlet openings 64c positioned on diametrically opposite sides of the end piece 62c. The enlarged inlet openings 64c are significantly larger than the inlet openings 62 typically found in a catheter (Figs. 2 and 3). Preferably, the cross-sectional size of each of the inlet openings 64c is significantly greater than the cross-sectional size of the interior passageway 56. In addition, a transverse dimension of each inlet opening 64c is preferably greater than one fourth of the circumferential distance around the main body 58 and the longitudinal dimension of each inlet opening 64c is greater than the transverse dimension. The enlarged size makes it difficult for blood clots to accumulate within the inlet openings 64c and to obstruct urine flow into and through the interior passageway 56 (Fig. 3). The rounded distal end 38 of the couvelaire tip 112 facilitates the insertion of the catheter 20c into the urinary canal 36.

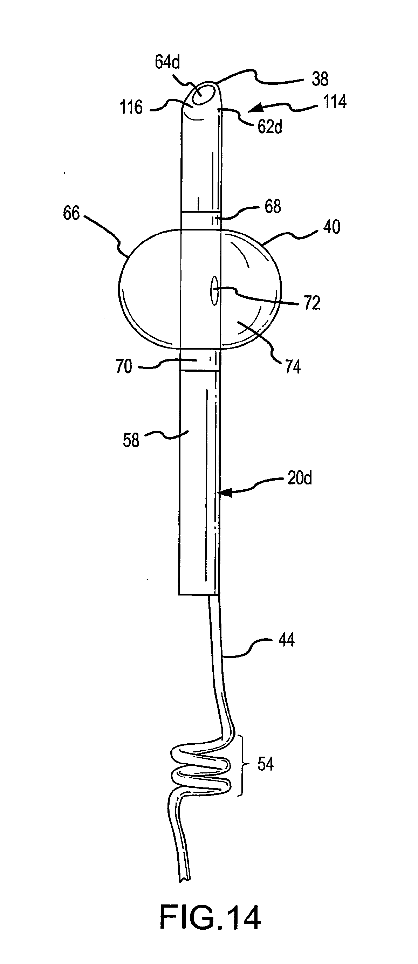

The indwelling catheter 20d shown in Fig. 14 has a beveled tip 114. The end piece 62d includes a somewhat flat beveled surface 116 that extends at an acute angle more transversely relative to the axis of the catheter from the distal end 38 of the end piece 62d than longitudinally. A single urine drainage inlet opening 64d is formed through the beveled surface 116. Because of the transverse angle of the beveled surface 116 relative to the axis of the catheter 20, the flow passageway through the inlet opening 64d more directly aligns with and enters the interior passageway 56 (Fig. 3). The single inlet opening 64d can be made of a size larger than the cross-sectional size of the passageway 56. The more-directly entering flow passageway through the inlet opening 64d avoids the right angle turn associated with the openings 62 in the end piece 64 (Figs. 2 and 3). The more-directly entering flow passageway through the inlet opening 64d provides less of an obstruction or restriction for the blood clots to accumulate and to obstruct the passageway 56. The end piece 62d retains a rounded distal end 38 which facilitates insertion of the catheter 20d into urinary canal 36. The beveled surface 116 also facilitates insertion of the catheter 20d into the urinary canal.

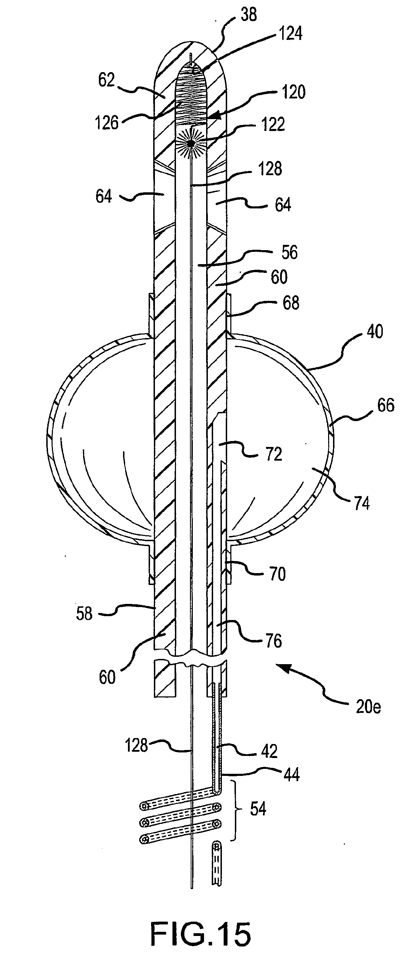

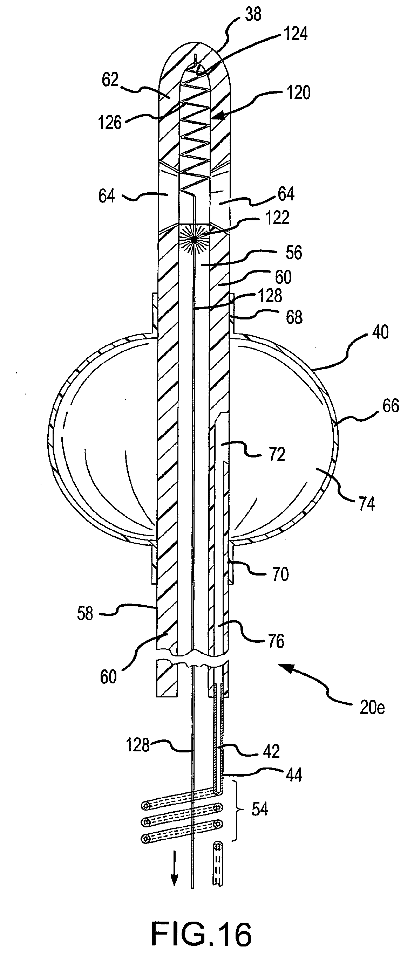

Obstructions from blood clots and other urinary tract matter can also be avoided by incorporating a clearing device 120 within the indwelling catheter 20e, as shown in Fig. 15. The clearing device 120 is located within the interior passageway 56 of the catheter 20e and includes an obstruction clearing contact element (e.g., brush structure 122) which is movable longitudinally along the interior passageway 56. The obstruction clearing contact element dislodges the blood clots or other obstructions within the passageway 56, or breaks the blood clots or obstructions into smaller pieces, or breaks open the accumulation of clots or obstructions in the openings 64, as a result of movement of the clot clearing contact element within the passageway 56, as shown in Fig. 16. Dislodging or breaking up the clots and obstructions opens the passageway 56 to the flow of urine and carries the obstructions out of the urinary tract with the flow of urine.

One form of the contact element of the clearing device 120 utilizes a brush structure 122 formed by a plurality of bristles held together to form the brush structure 122. Under normal use conditions, the brush structure 122 is retained in a stowed position at an internal distal end 124 of the passageway 56 within the end piece 62, spaced slightly distally from the inlet openings 64, as shown in Fig. 15. The brush structure 122 is held in the stowed position by bias force from a return spring 126 which extends between the brush structure 122 and the internal distal end 124 of the passageway 56. In its stowed position, the brush structure 122 does not interfere with the flow of urine through the inlet openings 64 and the interior passageway 56.

A thread-like tether 128 is attached to the brush structure 122 and the spring 126, and the tether 128 extends through the passageway 56 of the catheter 20e on the opposite side of the brush structure 122 from the return spring 126. When the catheter 20e is attached to the insertion tool 22 (Fig. 1), the tether 128 extends through the passageway 56 and the interior channel 88. of the insertion tool 22 (Fig. 10) and out of the handle 82 (Fig. 1). Once the indwelling catheter 20e has been inserted into the urinary canal 36 in its use final position within the bladder and the prostatic urethra (Fig. 7), the insertion tool 22 is disconnected from the indwelling catheter 20e and the insertion tool 22 is removed in the manner previously described. The tether 128 remains in position within the urinary canal 36 as the insertion tool 22 is removed, as a result of the tether 128 moving through the interior channel 88 of the insertion tool. The proximal end of the tether 128 remains exterior of the urinary canal 36.

When it is determined that the urine flow path through the passageway 56 is obstructed by blood clots, the brush structure 122 is moved out of the stowed position (Fig. 15) and is moved longitudinally proximally along the interior passageway 56 by pulling proximal end of the tether 128 at the exterior of the urinary canal 36, as shown in Fig. 16. The resiliency of the return spring 126 permits proximal longitudinal movement. As the brush structure 122 moves along the passageway 56, the blood clots or obstructions are contacted by the brush structure 122. The longitudinal movement of the brush structure dislodges and shears the clots out of the inlet openings 64 and the passageway 56. Deflected bristles of the brush structure 122 within the interior passageway 56 spring outwardly through the inlet openings 64 to push the clots or obstructions out of those inlet openings. The bristles of the brush structure 122 also shear or break the clots or obstructions within the inlet openings and the passageway 56 into smaller pieces. The dislodged, broken and smaller clots or obstructions flow with the urine through the opened passageway 56 and into the urinary canal 36, or the movement of the brush structure 122 pushes the clots or obstructions through the passageway 56 out of the catheter 20e. The clots or obstructions thereafter pass through the remaining portion of the urinary canal 36 and out of the body by the flow of urine. The tether 128 constitutes one form of an activation device for operating the obstruction clearing device 120.

After the brush structure 122 has cleared the clots from the inlet opening 64 and the passageway 56, the tether 128 is released and the return spring 126 pulls the brush structure 122 from the position shown in Fig. 16 back into the stowed position at the internal distal end 124 of the passageway 56 shown in Fig. 15. Thereafter, urine may again flow through the passageway 56 in the normal matter. Should the inlet opening 64 or the passageway 56 again become obstructed, the brush structure 122 can again be moved longitudinally by pulling on the tether 128 to again clear the urine flow path through the inlet openings 64 and the passageway 56. The clearing device 120 can be used as many times as needed in this manner to keep the catheter 20e open to the flow of urine.

An alternative form of the clearing device 120 provides for a one-time use to clear the urine flow passageway. In this circumstances, the return spring 126 is omitted. The brush structure 122 is retained in the stowed position (Fig. 15) by frictional forces between the bristles and the inside surface of the passageway 56 at the internal distal end 124. If the urine flow through the passageway 56 becomes obstructed, the tether 128 is pulled and the brush structure 122 is moved from the stowed position longitudinally through the passageway 56 (Fig. 16), thereby clearing the passageway of clots or obstructions. Continuing to pull the tether 128 removes the brush structure 122 from the interior passageway 56 and into the urinary canal 36. The clearing device 120 is then pulled completely out of the urinary canal 36.

In this way the obstruction is removed at the same time that the brush structure 122 is removed from within the indwelling catheter 20e. While the clearing device 120 can only be used once in this manner, a single use may prove satisfactory for those TUMT, heat treatment and other patients for whom blood clots and other obstructions are not expected to be a significant issue. Also, the use of the single-use clearing indwelling catheter 20e may be prescribed if the physician knows or prefers to replace the indwelling catheter 20e on a periodic basis during healing.

The clearing device 120 can be used while the insertion tool 22 remains connected to the indwelling catheter 20e for flushing the catheter 20. The tether 128 extends through the interior channel 88 in the insertion tool 22 and therefore does not interfere with the flow of flushing fluid through the interior channel 88 and into the interior passageway 56. Clearing the blood clots or obstructions by use of the clearing device 120 in combination with flushing or in alternation with flushing, may be reserved for those situations where flushing by itself is insufficient to eliminate the obstructions. Using the clearing device 120 can also enhance the effect of the flushing, by loosening and breaking the clots or obstructions prior to flushing. In this manner, the amount and extent of flushing may be reduced, which can be important in minimizing the discomfort associated with forcing additional flushing fluid into an already-filled bladder.

Forcing the flushing fluid into the interior channel 88 and interior passageway 56 may also move the brush structure 122 back to the stowed position. In this circumstance, the return spring 126 is not needed to obtain multiple uses of the clearing device 120. Forcing the flushing fluid into the interior channel 88 and interior passageway 56 may also help to move the brush structure 122 back to the stowed position, thereby assisting the return spring 126.

In those catheters having inlet openings 64 spaced slightly proximally of the distal most end 38 (e.g. Figs. 3, 12, 13), as represented in Fig. 15, the stowed position of the clearing device 120 is slightly distal of the most distal openings 64 within the interior passageway 56. In those catheters which have inlet openings that extend to or through the distal most end 38 (e.g. Figs. 11 and 14), the clearing device 120 may take a different form from the brush structure 122 or a part of the urine flow may normally pass through the brush structure 122. The different form of the clearing device 120 may permit it to move out of the path of the normal urine flow through the interior passageway 56 yet move across the inlet openings 64 and along the interior passageway 56 in such a way to clear obstructions within the inlet openings. This may be accomplished by rotating and longitudinally moving the clearing device 120 within the interior passageway 56, rather than solely moving the clearing device 120 longitudinally within the interior passageway 56. If the inlet openings 64 are sufficiently large, the exposed brush structure 122 may not substantially interfere with normal urine flow, because the size of the inlet opening 64 is so large that the presence of the brush structure 122 at a portion of the inlet openings 64 is not significant. Any clots which might form on the brush structure 120 itself are broken as the brush structure moves into the interior passageway 56, while still breaking, sharing and dislodging other obstructions present in the interior passageway.

Although the enlarged urinary inlet opening configurations of 64a-64d and the clearing device 120 have been shown in connection with a partial-length indwelling catheter, they are also applicable for use in full-length catheters. Use with a full-length catheter occurs in the same manner as occurs while the insertion tool 22 remains connected to the partial length catheter.

In the manner described, the partial-length catheter allows voluntary natural use of the external urinary sphincter muscle 34 to start and stop urine flow. The location of the indwelling catheter within the prostate gland 30 bypasses most of the urine flow from contact with the tissue of the prostate gland 30 which has been affected by the surgical procedure, thereby preventing or lessening pain and irritation. Any blood which may accumulate within the interior passageway of the indwelling catheter, and possibly cause obstructions due to clots, is flushed and cleared from the interior of the indwelling catheter prior to removal of the insertion tool 22. The separation of the insertion tool 22 from the indwelling catheter 20 may be delayed until the risks of obstructions from blood clots and the like has passed.

The indwelling catheter also assures a passageway for urine to flow through a prostate gland 30 that is diseased and swollen from BPH or other diseases. The catheter reliably remains in position for use due to the anchoring effects of the inflated balloon 40 and the coiled section 54, thereby lessening the risk of further surgical procedures to reposition and remove an inadvertently displaced catheter. Obstructions blocking the urine flow into and through the catheter, such as blood clots, are reduced or eliminated by the low friction coating and the various configurations of the inlet openings 64 at the distal end of the catheter. If the interior passageway 56 does become obstructed, the blood clot or other obstruction clearing device 120 is selectively movable within the passageway 56 to eliminate the obstruction without heaving to resort to a surgical procedure for replacing the catheter 20. The partial-length indwelling catheter also preserves natural control over the urine flow in the normal manner by the external urinary sphincter muscle.

Presently preferred embodiments of the invention and many of its improvements have been described above with a degree of particularity. The description is of preferred examples for implementing the invention, and is not necessarily intended to limit the scope of the invention. The scope of the invention is defined by the following claims.

## Claims

1. An indwelling catheter (20) to drain urine from a bladder (32) to a location adjacent to an external urinary sphincter muscle (34) in a urinary tract (26) which also includes a urinary canal (36) extending from the external urinary sphincter muscle to an exterior opening, comprising:
a main body (58) having a distal end (38), a proximal end and a length sufficient to position the distal end within the bladder and to position the proximal end adjacent to and distal of the sphincter muscle within the urinary tract, the main body defining a urine drainage interior passageway (56) extending from the distal end to the proximal end, and at least one inlet opening (64; 64a; 64b; 64c; 64d) extending through the distal end of the main body into the urine drainage interior passageway (56);
a balloon (40) attached to the distal end (38) of the main body, the balloon expandable in size within the bladder to maintain the distal end in the bladder and restrain the main body against proximal movement within the urinary tract from a use position, the use position locating the distal end of the main body in the bladder and the proximal end of the main body adjacent to and distal of the external urinary sphincter muscle;
an inflation tube (44) having a distal end, a proximal end and a length extending between the distal end proximal ends, the distal end connected to the main body, the length sufficient to extend from the main body through the urinary canal to the exterior opening when the main body is in the use position, the inflation tube and the main body defining an inflation passageway extending from the proximal end of the inflation tube to the balloon through which to deliver inflation fluid for expanding the balloon; and
**characterised by** a coiled section (54) of the inflation tube formed at a position along the inflation tube to locate the coiled section within the urinary canal adjacent to and proximal of the sphincter muscle when the main body is located in the use position, the coiled section being suited for interacting with a constriction of the urinary tract by the external urinary sphincter muscle to restrain the main body against distal movement within the urinary tract from the use position.

2. An indwelling catheter as defined in claim 1, wherein:
the coiled section (54) comprises a plurality of individual adjacent resilient coils each formed by the inflation tube.

3. An indwelling catheter as defined in claim 1, wherein:
the coiled section has a center opening and an outer transverse dimension, the main body has an outer transverse dimension, and the outer transverse dimension of the coiled section is at least equal to the outer transverse dimension of the main body.

4. An indwelling catheter as defined in claim 1, wherein:
the coiled section is resilient in both, a transverse dimension and a longitudinal dimension.

5. An indwelling catheter as defined in claim 1, wherein:
at least one urine inlet opening extends through the distal end of the main body and into the interior passageway, each inlet opening having a cross sectional size, the cumulative cross-sectional size of all inlet openings in the distal end being greater than the cross-sectional size of the interior passageway to facilitate passing urine and blood clots from the bladder through the interior passageway and into the urinary canal.openings.

6. An indwelling catheter as defined in claim 1, wherein:
the distal end (38) of the main body includes a rounded tip;
a single inlet opening (64a) extends through the distal end of the main body;
the single inlet opening (64a) has a predetermined configuration defined by the absence of a longitudinal half portion of the rounded tip and the distal end adjacent to the rounded tip, the longitudinal half portion defined substantially by a longitudinal bisection of the rounded tip and the portion of the distal end adjoining the rounded tip; and
the single inlet opening and a remaining longitudinal half portion of the rounded tip and the distal end establish a whistle-tip shape of the distal end of the main body.

7. An indwelling catheter as defined in claim 1, wherein:
the distal end (38) of the main body includes a rounded tip;
a plurality of inlet openings (64b) extend through the distal end of the main body; and
each of the inlet openings (64b) is longitudinally and circumferentially spaced from an adjacent inlet opening along the portion of the distal end adjacent to the rounded tip.

8. An indwelling catheter as defined in claim 1, wherein:
the distal end (38) of the main body includes a rounded tip;
two inlet openings (64c) extend through the distal end of the main body;
the two inlet openings are positioned radially opposite one another on the distal portion of the main body adjacent to the rounded tip, each of the two inlet openings has a cross-sectional size, and the cross-sectional size of each of the two inlet openings is substantially greater than the cross-sectional size of the interior passageway;
each of the two inlet openings has a circumferential dimension greater than one-fourth of the circumferential distance around the main body;
each of the two inlet openings is elongated with a longer dimension of each elongated opening extending longitudinally along the distal end; and
the two inlet openings define a couvelaire tip configuration of the distal end of the main body.

9. An indwelling catheter as defined in claim 1, wherein:
the distal end (38) of the main body includes a beveled surface which extends substantially transversely at an acute angle relative to a longitudinal axis through the distal end;
a single inlet opening (64d) extends through the distal end of the main body; and
the single inlet opening extends through the beveled surface substantially directly into the interior passageway of the main body.

10. An indwelling catheter as defined in claim 1, in combination with:
an insertion tool (22) for connection to the main body (58) to move the indwelling catheter (20) within the urinary tract to the use position, the insertion tool having first and second opposite ends and a length sufficient to position the first end within the urinary tract distal of the sphincter muscle while the second end is at the exterior of the urinary canal; and wherein:
the insertion tool (22) extends through the center opening of the coiled section (54) when the insertion tool is connected to the indwelling catheter.

11. An indwelling catheter as defined in claim 10, further comprising:
a separable connection (52) between the main body and the insertion tool.

12. An indwelling catheter as defined in claim 11, wherein:
the separable connection (52) connects the main body (58) to the insertion tool (22) for movement of the insertion tool and the indwelling catheter as a unit when positioning the indwelling catheter in the use position; and
the separable connection (52) permits separation of the main body from the insertion tool in response to continued proximal movement of the insertion tool when the expanded balloon restrains the main body against proximal movement from the use position.

13. An indwelling catheter as defined in claim 10, wherein:
the insertion tool defines an interior channel extending between the first and second opposite ends of the insertion tool; and
the interior channel of the insertion tool is in fluid communication with the interior passageway of the main body when the insertion tool is connected to the indwelling catheter at the separable connection.

14. An indwelling catheter as defined in claim 10, wherein:
the dimensions of the coiled section (54) and the insertion tool (22) are such that the coiled section (54) is loosely wound around the insertion tool (22), thereby allowing the insertion tool (22) to be withdrawn through a center of the coiled section (54) as the insertion tool (22) is disconnected from the indwelling catheter (20).

15. An indwelling catheter as defined in claim 10, wherein:
the separable connection (52) includes a selectively disconnectable bridging structure (90) extending between the main body and the insertion tool, the bridging structure fastening the main body to the insertion tool when connected, the bridging structure (90) releasing the main body from the insertion tool when the bridging structure is disconnected to permit separation of the indwelling catheter from the insertion tool in response to continued proximal movement of the insertion tool when the expanded balloon restrains the main body against proximal movement from the use position.

16. An indwelling catheter as defined in claim 15, wherein:
the selectively disconnectable bridging structure (90) comprises a cord which extends between the main body and the insertion tool when the bridging structure connects the main body to the bridging tool; and
the extension of the cord between the main body and the insertion tool is eliminated when the bridging structure is disconnected.

17. An indwelling catheter as defined in claim 1, further comprising:
a clearing device (120) positioned within the interior passageway and including a contact element (122) movable within the interior passageway past the at least one inlet opening to contact and disintegrate and facilitate movement of blood clots and obstructions within the one inlet opening and the interior passageway with the flow of urine through the interior passageway.

18. An indwelling catheter as defined in claim 17, wherein
the contact element (122) is normally positioned within the interior passageway in a stowed position between each inlet opening and a distal end of the interior passageway; and
the contact element (122) is movable from the stowed position to a position proximal of each inlet opening in the interior passageway through the main body.

19. An indwelling catheter as defined in claim 17, further comprising:
an activation device (128) connected to the contact element (122) and extending through the urinary canal to a position outside an exterior opening of the urinary canal; and wherein:
the contact element (122) is selectively moved by manipulation of the activation device (128) at the position outside the exterior opening of the urinary canal.

20. A catheter as defined in claim 19, further comprising in combination:
an insertion tool (22) for inserting the catheter into the urinary tract to the use position, the insertion tool having a distal end for connecting with the proximal end of the main body and a proximal end that extends to a position outside the exterior opening of the urinary canal, the insertion tool having an interior channel extending from its distal end to its proximal end, the interior channel communicating with the interior passageway in the main body when the distal end of the insertion tool is connected to the proximal end of the main body; and wherein:
the activation device (128) extends through the interior channel of the insertion tool when the insertion tool is connected to the main body, and the insertion tool extends through the center opening of the coiled section when the insertion tool is connected to the indwelling catheter.

21. A catheter as defined in claim 20, wherein:
the contact element is retained within the interior passageway for removal and transportation through the interior passageway and out of the main body by manipulation of the activation device at the position outside the exterior opening of the urinary canal.

22. A catheter as defined in claim 17, further comprising:
a return element (126) connected between the contact element and the main body to bias the contact element into an initial position from which the contact element commences movement; and
the return element (126) moving the contact element back to the initial position after the contact element has been moved past the inlet opening and along the interior passageway.

23. An indwelling catheter as defined in claim 22, wherein:
the return element (126) comprises a spring.

24. A catheter as defined in claim 19, wherein:
the activation device (128) comprises a tether connected to the contact element (122) to move the contact element past the inlet opening and along the interior passageway upon pulling the tether at the position outside the exterior opening of the urinary canal.

25. A catheter as defined in claim 17, wherein:
the contact element (122) is retained within the interior passageway for removal and transportation through the interior passageway and out of the main body by pulling the tether after moving the contact element past the inlet opening.

26. A catheter as defined in claim 17, wherein:
the contact element comprises a brush structure.

27. A catheter as defined in claim 26, wherein:
the brush structure includes a plurality of bristles.

28. A catheter as defined in claim 27, wherein:
the brush structure is maintained in the interior passageway by friction between the bristles and the interior passageway until the contact member is moved.

29. A catheter as defined in claim 1, further comprising:
a low friction coating extending over the distal end of the main body, within each inlet opening, and along the interior passageway.

## Patentansprüche

1. Verweilkatheter (Dauerkatheter) (20) zum Ableiten von Urin aus einer Blase (32) an einen zu einem außenliegenden Blasenschließmuskel (34) benachbarten Ort in einem Harntrakt (Harnweg) (26), welcher auch eine sich vom außenliegenden Blasenschließmuskel zu einer äußeren Öffnung erstreckende Harnröhre (36) umfasst, aufweisend:
einen Hauptkörper (58) mit einem distalen Ende (38), einem proximalen Ende und einer Länge, welche ausreicht, um das distale Ende in der Blase zu positionieren und das proximale Ende benachbart und distal zu dem Blasenschließmuskel im Harntrakt zu positionieren, wobei der Hauptkörper einen inneren Durchgang (56) zur Urinableitung, welcher sich vom distalen zum proximalen Ende erstreckt, definiert, und mindestens eine Einlassöffnung (64; 64a; 64b; 64c; 64d), welche sich durch das distale Ende des Hauptkörpers in den inneren Durchgang (56) zur Urinableitung erstreckt;
einen Ballon (40), angebracht am distalen Ende (38) des Hauptkörpers, wobei der Ballon in der Blase expandiert werden kann, um das distale Ende in der Blase zu halten und den Hauptkörper von proximaler Bewegung im Harntrakt aus der Verwendungsposition heraus abzuhalten, wobei in der Verwendungsposition das distale Ende des Hauptkörpers in der Blase und das proximale Ende des Hauptkörpers benachbart und distal zu dem außenliegenden Blasenschließmuskel gelegen ist;
ein Aufblasrohr (44) mit einem distalen Ende, einem proximalen Ende und einer sich zwischen dem distalen und proximalen Ende erstreckenden Länge, wobei das distale Ende mit dem Hauptkörper verbunden ist, wobei die Länge ausreicht, um sich vom Hauptkörper durch die Harnröhre zur äußeren Öffnung zu erstrecken, wenn sich der Hauptkörper in der Verwendungsposition befindet, wobei das Aufblasrohr und der Hauptkörper einen Befüllungsgang definieren, welcher sich vom proximalen Ende des Aufblasrohrs zu dem Ballon erstreckt, durch welchen eine Befüllungsflüssigkeit zur Expandierung des Ballons geleitet werden kann; und
**gekennzeichnet durch** einen spulenförmigen Abschnitt (54) des Aufblasrohrs, welcher an einer Position entlang dem Aufblasrohr gebildet wird, um den spulenförmigen Abschnitt in der Harnröhre benachbart und proximal zu dem Blasenschließmuskel zu positionieren, wenn sich der Hauptkörper in der Verwendungsposition befindet, wobei der spulenförmige Abschnitt geeignet ist, um mit einer Einschnürung des Harntraktes **durch** den außenliegenden Blasenschließmuskel in Wechselwirkung zu treten, um den Hauptkörper von distaler Bewegung im Harntrakt aus der Verwendungsposition heraus abzuhalten.

2. Verweilkatheter nach Anspruch 1, wobei:
der spulenförmige Abschnitt (54) eine Vielzahl von einzelnen, benachbarten elastischen Windungen, welche jeweils durch das Aufblasrohr gebildet werden, umfasst.

3. Verweilkatheter nach Anspruch 1, wobei:
der spulenförmige Abschnitt eine zentrale Öffnung und eine äußere, querlaufende Erstreckung besitzt;
der Hauptkörper eine äußere, querlaufende Erstreckung besitzt; und
die äußere querlaufende Erstreckung des spulenförmigen Abschnitts mindestens gleich der äußeren querlaufenden Erstreckung des Hauptkörpers ist.

4. Verweilkatheter nach Anspruch 1, wobei:
der spulenförmige Abschnitt sowohl in einer querverlaufenden Erstreckung als auch in einer längsverlaufenden (longitudinalen) Erstreckung elastisch ausgebildet ist.

5. Verweilkatheter nach Anspruch 1, wobei:
mindestens eine Urineinlassöffnung sich durch das distale Ende des Hauptkörpers und in den inneren Durchgang erstreckt, wobei jede Einlassöffnung eine Querschnittsfläche besitzt, wobei die kumulative Querschnittsfläche aller Einlassöffnungen in dem distalen Ende größer ist als die Querschnittsfläche des inneren Durchgangs, um den Durchtritt von Urin und Blutverklumpungen aus der Blase durch den inneren Durchgang und in die Öffnungen der Harnröhre zu ermöglichen.

6. Verweilkatheter nach Anspruch 1, wobei:
das distale Ende (38) des Hauptkörpers eine abgerundete Spitze aufweist;
sich eine einzelne Einlassöffnung (64a) durch das distale Ende des Hauptkörpers erstreckt;
die einzelne Einlassöffnung (64a) eine vorbestimmte Gestalt, welche durch die Abwesenheit eines längsverlaufenden halben Abschnitts der abgerundeten Spitze und des benachbart zur abgerundeten Spitze gelegenen distalen Endes definiert ist, wobei der längsverlaufende halbe Abschnitt im Wesentlichen durch eine longitudinale Bisektion der abgerundeten Spitze und des Abschnitts des distalen Endes, welcher der abgerundeten Spitze benachbart ist, definiert wird; und
die einzelne Einlassöffnung und ein verbleibender, längsverlaufender halber Abschnitt der abgerundeten Spitze und des distalen Endes eine pfeifenartige Gestalt des distalen Endes des Hauptkörpers bilden.

7. Verweilkatheter nach Anspruch 1, wobei:
das distale Ende (38) des Hauptkörpers eine abgerundete Spitze beinhaltet;
eine Vielzahl von Einlassöffnungen (64b) sich durch das distale Ende des Hauptkörpers erstrecken; und
jede der Einlassöffnungen (64b) in Längsrichtung und in Richtung des Umfangs entlang dem zur abgerundeten Spitze benachbart gelegenen Abschnitt des distalen Endes von einer benachbarten Einlassöffnung beabstandet ist.

8. Verweilkatheter nach Anspruch 1, wobei:
das distale Ende (38) des Hauptkörpers eine abgerundete Spitze aufweist;
zwei Einlassöffnungen (64c) sich durch das distale Ende des Hauptkörpers erstrecken;
die zwei Einlassöffnungen einander radial entgegengesetzt auf dem distalen Abschnitt des Hauptkörpers, welcher benachbart zur abgerundeten Spitze gelegen ist, positioniert sind, wobei jede der zwei Einlassöffnungen eine Querschnittsfläche besitzt und wobei die Querschnittsfläche einer jeden der beiden Einlassöffnungen im Wesentlichen größer ist als die Querschnittsfläche des inneren Durchgangs;
jede der beiden Einlassöffnungen eine Ausdehnung des Umfangs von größer als einem Viertel des Umfangs des Hauptkörpers besitzt;
jede der beiden Einlassöffnungen gestreckt ist mit einer größeren Ausdehnungsrichtung einer jeden gestreckten Öffnung in Längsrichtung zum distalen Ende; und
die zwei Einlassöffnungen die Gestalt einer Couvelaire-Spitze des distalen Endes des Hauptkörpers definieren.

9. Verweilkatheter nach Anspruch 1, wobei:
das distale Ende (38) des Hauptkörpers eine abgeschrägte Oberfläche aufweist, welche sich im Wesentlichen in einem spitzen Winkel querverlaufend in Relation zu einer Längsachse durch das distale Ende erstreckt; und
sich eine einzelne Einlassöffnung (64d) durch das distale Ende des Hauptkörpers erstreckt; und
sich die einzelne Einlassöffnung durch die abgeschrägte Oberfläche im Wesentlichen direkt in den inneren Durchgang des Hauptkörpers erstreckt.

10. Verweilkatheter nach Anspruch 1, in Kombination mit:
einem Einführungsinstrument (22) zur Verbindung mit dem Hauptkörper (58), um den Verweilkatheter (20) im Harntrakt in die Verwendungsposition zu bewegen, wobei das Einfiihrungsinstrument erste und zweite gegenüberliegende Enden und
eine Länge, welche ausreicht, um das erste Ende im Harntrakt distal des Schließmuskels zu positionieren, während sich das zweite Ende außerhalb der Harnröhre befindet, besitzt; und wobei:
sich das Einführungsinstrument (22) durch die zentrale Öffnung des spulenförmigen Abschnitts (54) erstreckt, wenn das Einführungsinstrument mit dem Verweilkatheter verbunden ist.

11. Verweilkatheter nach Anspruch 10, weiterhin umfassend:
eine trennbare Verbindung (52) zwischen dem Hauptkörper und dem Einführungsinstrument.

12. Verweilkatheter nach Anspruch 11, wobei:
die trennbare Verbindung (52) den Hauptkörper (58) mit dem Einführungsinstrument (22) verbindet, um das Einführungsinstrument und den Verweilkatheter als Einheit zu bewegen, wenn der Verweilkatheter in die Verwendungsposition gebracht wird; und
die trennbare Verbindung (52) die Trennung des Hauptkörpers von dem Einführungsinstrument als Reaktion auf eine fortgesetzte proximale Bewegung des Einführungsinstruments erlaubt, wenn der expandierte Ballon den Hauptkörper von proximaler Bewegung aus der Verwendungsposition heraus abhält.

13. Verweilkatheter nach Anspruch 10, wobei:
das Einführungsinstrument einen inneren Kanal definiert, welcher sich zwischen den ersten und zweiten entgegengesetzten Enden des Einführungsinstruments erstreckt; und
der innere Kanal des Einführungsinstruments über eine Flüssigkeit mit dem inneren Durchgang des Hauptkörpers kommuniziert, wenn das Einführungsinstrument mit dem Verweilkatheter über die trennbare Verbindung verbunden ist.

14. Verweilkatheter nach Anspruch 10, wobei:
die Ausdehnungen des spulenförmigen Abschnitts (54) und des Einführungsinstruments (22) derart sind, dass der spulenförmige Abschnitt (54) lose (locker) um das Einführungsinstrument (22) gewunden ist, wodurch das Einführungsinstrument (22) durch eine Mitte des spulenförmigen Abschnitts (54) entfernt werden kann, wenn das Einführungsinstrument (22) von dem Verweilkatheter (20) getrennt wird.

15. Verweilkatheter nach Anspruch 10, wobei:
die trennbare Verbindung (52) eine selektiv bzw. unabhängig trennbare verbrückende Struktur (90) aufweist, welche sich zwischen dem Hauptkörper und dem Einführungsinstrument erstreckt, wobei die verbrückende Struktur den Hauptkörper an dem Einführungsinstrument befestigt, wenn sie verbunden ist, wobei die verbrückende Struktur (90) den Hauptkörper von dem Einführungsinstrument löst, wenn die verbrückende Struktur getrennt ist, um die Trennung des Verweilkatheters von dem Einführungsinstrument als Reaktion auf eine fortgesetzte proximale Bewegung des Einführungsinstruments zu ermöglichen, wenn der expandierte Ballon den Hauptkörper von proximalen Bewegung aus der Verwendungsposition heraus abhält.

16. Verweilkatheter nach Anspruch 15, wobei:
die selektiv bzw. unabhängig trennbare verbrückende Struktur (90) eine Schnur umfasst, welche sich zwischen dem Hauptkörper und dem Einführungsinstrument erstreckt, wenn die verbrückende Struktur den Hauptkörper mit dem verbrückenden Mittel verbindet; und
die Erstreckung der Schnur zwischen dem Hauptkörper und dem Einführungsinstrument aufgehoben ist, wenn die verbrückende Struktur getrennt ist.

17. Verweilkatheter nach Anspruch 1, weiterhin umfassend:
eine Freihalteeinrichtung (Klärungseinrichtung) (120), welches in dem inneren Durchgang angebracht ist und ein Kontaktelement (122) aufweist, welche in dem inneren Durchgang über die mindestens eine Einlassöffnung hinaus beweglich ist, um mit Blutverklumpungen und Verstopfungen in Kontakt zu treten und diese aufzulösen und eine Bewegung von Blutverklumpungen und Verstopfungen in der einen Einlassöffnung und dem inneren Durchgang mit dem Fluss des Urins durch den inneren Durchgang zu ermöglichen.

18. Verweilkatheter nach Anspruch 17, wobei:
das Kontaktelement (122) üblicherweise in dem inneren Durchgang in einer Lagerposition zwischen jeder einzelnen Einlassöffnung und dem distalen Ende des inneren Durchgangs befindlich ist; und
das Kontaktelement (122) aus der Lagerposition zu einer Position proximal von jeder Einlassöffnung in dem inneren Durchgang durch den Hauptkörper bewegbar ist.

19. Verweilkatheter nach Anspruch 17, weiterhin umfassend:
eine Aktivierungseinrichtung (128), welche mit dem Kontaktelement (122) verbunden ist und sich durch die Harnröhre zu einer Position außerhalb einer äußeren Öffnung der Harnröhre erstreckt; und wobei:
das Kontaktelement (122) selektiv bzw. unabhängig durch Betätigung der Aktivierungseinrichtung (128) an der Position außerhalb der äußeren Öffnung der Harnröhre bewegt werden kann.

20. Katheter nach Anspruch 19, weiterhin umfassend in Kombination:
ein Einführungsinstrument (22) zur Einführung des Katheters in den Harntrakt bis zur Verwendungsposition, wobei das Einführungsinstrument ein distales Ende zur Verbindung mit dem proximalen Ende des Hauptkörpers und ein proximales Ende, welches sich zu einer Position außerhalb der äußeren Öffnung der Harnröhre erstreckt, besitzt, wobei das Einführungsinstrument einen sich von seinem distalen zu seinem proximalen Ende erstreckenden inneren Kanal besitzt, wobei der innere Kanal mit dem inneren Durchgang in dem Hauptkörper kommuniziert, wenn das distale Ende des Einführungsinstruments mit dem proximalen Ende des Hauptkörpers verbunden ist; und wobei:
die Aktivierungseinrichtung (128) sich durch den inneren Kanal des Einführungsinstruments erstreckt, wenn das Einführungsinstrument mit dem Hauptkörper verbunden ist, und wobei sich das Einführungsinstrument durch die zentrale Öffnung des spulenförmigen Abschnitts erstreckt, wenn das Einführungsinstrument mit dem Verweilkatheter verbunden ist.

21. Katheter nach Anspruch 20, wobei:
das Kontaktelement in dem inneren Durchgang zurückgehalten wird zu Zwecken der Entfernung und des Transports durch den inneren Durchgang und aus dem Hauptkörper heraus durch Betätigung der Aktivierungseinrichtung an einer Position außerhalb der äußeren Öffnung der Harnröhre.

22. Katheter nach Anspruch 17, weiterhin umfassend:
ein Rückstellelement (126), welches zwischen dem Kontaktelement und dem Hauptkörper angeordnet und mit diesen verbunden ist, um das Kontaktelement in eine Ausgangsposition zu bringen, aus welcher das Kontaktelement mit der Bewegung beginnt; und
das Rückstellelement (126) das Kontaktelement zurück in eine Ausgangsposition bewegt, nachdem das Kontaktelement über die Einlassöffnung hinaus und entlang dem inneren Durchgang bewegt wurde.

23. Verweilkatheter nach Anspruch 22, wobei:
das Rückstellelement (126) eine Feder umfasst.

24. Katheter nach Anspruch 19, wobei:
die Aktivierungseinrichtung (128) eine Halteschnur umfasst, welche mit dem Kontaktelement (122) verbunden ist, um das Kontaktelement über die Einlassöffnung hinaus und entlang dem inneren Durchgang zu bewegen, wenn an der Halteschnur an einer Position außerhalb der äußeren Öffnung der Harnröhre gezogen wird.

25. Katheter nach Anspruch 17, wobei:
das Kontaktelement (122) in dem inneren Durchgang zurückgehalten wird zu Zwecken der Entfernung und des Transports durch den inneren Durchgang und aus dem Hauptkörper heraus durch Ziehen an der Halteschnur, nachdem das Kontaktelement an der Einlassöffnung vorbeibewegt wurde.

26. Katheter nach Anspruch 17, wobei:
das Kontaktelement eine Bürstenstruktur umfasst.

27. Katheter nach Anspruch 26, wobei:
die Bürstenstruktur eine Vielzahl von Borsten aufweist.

28. Katheter nach Anspruch 27, wobei:
die Bürstenstruktur in dem inneren Durchgang durch Reibung zwischen den Borsten und dem inneren Durchgang beibehalten wird, bis das Kontaktmittel bewegt wird.

29. Katheter nach Anspruch 1, weiterhin umfassend:
eine Beschichtung mit niedriger Reibung, welche sich über das distale Ende des Hauptkörpers, innerhalb einer jeden Einlassöffnung, und entlang dem inneren Durchgang erstreckt.

## Revendications

1. Cathéter à demeure (20) pour drainer l'urine d'une vessie (32) vers un endroit adjacent à un muscle sphincter urinaire externe (34) dans les voies urinaires (26), qui comprend également un canal urinaire (36) s'étendant depuis le muscle sphincter urinaire externe vers une ouverture extérieure, comprenant :
un corps principal (58) ayant une extrémité distale (38), une extrémité proximale et une longueur suffisante pour positionner l'extrémité distale dans la vessie et pour positionner l'extrémité proximale de manière adjacente et distale par rapport au muscle sphincter dans les voies urinaires, le corps principal définissant un passage intérieur de drainage de l'urine (56) s'étendant depuis l'extrémité distale vers l'extrémité proximale, et au moins une ouverture d'entrée (64, 64a, 64b, 64c, 64d) s'étendant à travers l'extrémité distale du corps principal dans le passage intérieur de drainage d'urine (56),
un ballonnet (40) fixé à l'extrémité distale (38) du corps principal, le ballonnet dilatable en taille dans la vessie, pour maintenir l'extrémité distale dans la vessie et limiter le corps principal contre le mouvement proximal dans les voies urinaires d'une position d'utilisation, la position d'utilisation positionnant l'extrémité distale du corps principal dans la vessie et l'extrémité proximale du corps principal de manière adjacente et distale par rapport au muscle sphincter urinaire externe,
un tube de gonflage (44) ayant une extrémité distale, une extrémité proximale et une longueur s'étendant entre les extrémités proximales de l'extrémité distale, l'extrémité distale reliée au corps principal, la longueur suffisante pour s'étendre du corps principal, à travers le canal urinaire vers l'ouverture extérieure quand le corps principal est dans la position d'utilisation, le tube de gonflage et le corps principal définissant un passage de gonflage s'étendant de l'extrémité proximale du tube de gonflage vers le ballonnet à travers lequel il est possible de fournir un fluide de gonflage pour dilater le ballonnet,
**caractérisé par** une section à bobine (54) du tube de gonflage formé dans une position le long du tube de gonflage, afin de localiser la section à bobine dans le canal urinaire adjacent et proximal par rapport au muscle sphincter, quand le corps principal est situé dans la position d'utilisation, la section à bobine étant adaptée pour interagir avec une constriction des voies urinaires par le biais du muscle sphincter urinaire externe pour restreindre le corps principal contre le mouvement distal dans les voies urinaires depuis la position d'utilisation.

2. Cathéter à demeure selon la revendication 1, dans lequel :
la section à bobine (54) comprend une pluralité de bobines résilientes adjacentes formées par le tube de gonflage.

3. Cathéter à demeure selon la revendication 1, dans lequel :
la section à bobine a une ouverture centrale et une dimension transversale extérieure, le corps principal a une dimension transversale extérieure, et la dimension transversale extérieure de la section à bobine est au moins égale à la dimension transversale extérieure du corps principal.

4. Cathéter à demeure selon la revendication 1, dans lequel :
la section à bobine est résiliente dans une dimension transversale et une dimension longitudinale.

5. Cathéter à demeure selon la revendication 1, dans lequel :
au moins une ouverture d'entrée d'urine s'étend à travers l'extrémité distale du corps principal et dans le passage intérieur, chaque ouverture d'entrée ayant une taille transversale, la taille transversale cumulative de toutes les ouvertures d'entrée dans l'extrémité distale étant supérieure à la taille transversale du passage intérieur, pour faciliter le passage de l'urine et des caillots de sang de la vessie à travers le passage intérieur et dans les ouvertures de canal urinaire.

6. Cathéter à demeure selon la revendication 1, dans lequel :
l'extrémité distale (38) du corps principal comprend une pointe arrondie,
une ouverture d'entrée unique (64a) s'étend à travers l'extrémité distale du corps principal,
l'ouverture d'entrée unique (64a) a une configuration prédéterminée définie par l'absence d'une demi-portion longitudinale de la pointe arrondie et l'extrémité distale adjacente à la pointe arrondie, la demi-portion longitudinale définie sensiblement par une bi-section longitudinale de la pointe arrondie et de la partie de l'extrémité distale joignant la pointe arrondie, et
l'ouverture d'entrée unique et une demi-portion longitudinale restante de la pointe arrondie et de l'extrémité distale établissent une forme de pointe de sifflet de l'extrémité distale du corps principal.

7. Cathéter à demeure selon la revendication 1, dans lequel :
l'extrémité distale (38) du corps principal comprend une pointe arrondie,
une pluralité d'ouvertures d'entrée (64b) s'étend à travers l'extrémité distale du corps principal et
chacune des ouvertures d'entrée (64b) est longitudinalement et circonférentiellement espacée d'une ouverture d'entrée adjacente, le long de la partie de l'extrémité distale adjacente à la pointe arrondie.

8. Cathéter à demeure selon la revendication 1, dans lequel :
l'extrémité distale (38) du corps principal comprend une pointe arrondie,
deux ouvertures d'entrée (64c) s'étendent à travers l'extrémité distale du corps principal,
les deux ouvertures d'entrée sont positionnées radialement à l'opposé l'une de l'autre sur la partie distale du corps principal, adjacent à la pointe arrondie, chacune des deux ouvertures d'entrée a une taille transversale, et la taille transversale de chacune des deux ouvertures d'entrée est sensiblement supérieure à la taille transversale du passage intérieur,
chacune des deux ouvertures d'entrée a une dimension circonférentielle supérieure à un quart de la distance circonférentielle autour du corps principal,
chacune des deux ouvertures d'entrée est allongée avec une dimension plus longue de chaque ouverture allongée s'étendant longitudinalement le long de l'extrémité distale et
les deux ouvertures d'entrée définissent une configuration de pointe de Couvelaire de l'extrémité distale du corps principal.

9. Cathéter à demeure selon la revendication 1, dans lequel :
l'extrémité distale (38) du corps principal comprend une surface biseautée qui s'étend sensiblement transversalement selon un angle aigu relativement à un axe longitudinal à travers l'extrémité distale,
une ouverture d'entrée unique (64d) s'étend à travers l'extrémité distale du corps principal et
l'ouverture d'entrée unique s'étend à travers la surface biseautée sensiblement directement dans le passage intérieur du corps principal.

10. Cathéter à demeure selon la revendication 1, combiné à :
un outil d'insertion (22) à connecter au corps principal (58) pour déplacer le cathéter à demeure (20) dans les voies urinaires vers la position d'utilisation, l'outil d'insertion ayant une première et une seconde extrémités opposées et une longueur suffisante pour positionner la première extrémité dans les voies urinaires, de manière distale par rapport au muscle sphincter tandis que la seconde extrémité est à l'extérieur du canal urinaire et dans lequel :
l'outil d'insertion (22) s'étend à travers l'ouverture centrale de la section à bobine (54) quand l'outil d'insertion est connecté au cathéter à demeure.

11. Cathéter à demeure selon la revendication 10, comprenant :
une connexion séparable (52) entre le corps principal et l'outil d'insertion.

12. Cathéter à demeure selon la revendication 11, dans lequel :
la connexion séparable (52) relie le corps principal (58) à l'outil d'insertion (22) pour un mouvement de l'outil d'insertion et du cathéter à demeure comme une unité lors du positionnement du cathéter à demeure dans la position d'utilisation et
la connexion séparable (52) permet la séparation du corps principal de l'outil d'insertion, en réponse à un mouvement proximal continu de l'outil d'insertion quand le ballonnet dilaté limite le corps principal contre le mouvement proximal depuis la position d'utilisation.

13. Cathéter à demeure selon la revendication 10, dans lequel :
l'outil d'insertion définit un canal intérieur s'étendant entre la première et la seconde extrémités opposées de l'outil d'insertion et
le canal intérieur de l'outil d'insertion est en communication fluidique avec le passage intérieur du corps principal quand l'outil d'insertion est relié au cathéter à demeure au niveau de la connexion séparable.

14. Cathéter à demeure selon la revendication 10, dans lequel :
les dimensions de la section à bobine (54) et de l'outil d'insertion (22) sont telles que la section à bobine (54) est enroulée de manière lâche autour de l'outil d'insertion (22), en alignant ainsi l'outil d'insertion (22) à retirer à travers un centre de la section à bobine (54) quand l'outil d'insertion (22) est déconnecté du cathéter à demeure (20).

15. Cathéter à demeure selon la revendication 10, dans lequel :
la connexion séparable (52) comprend une structure de pontage sélectivement déconnectable (90) s'étendant entre le corps principal et l'outil d'insertion, la structure de pontage fixant le corps principal à l'outil d'insertion quand il est connecté, la structure de pontage (90) libérant le corps principal de l'outil d'insertion quand la structure de pontage est déconnectée, pour permettre la séparation du cathéter à demeure de l'outil d'insertion, en réponse à un mouvement proximal continu de l'outil d'insertion, quand le ballonnet dilaté limite le corps principal contre le mouvement proximal depuis la position d'utilisation.

16. Cathéter à demeure selon la revendication 15, dans lequel :
la structure de pontage sélectivement déconnectable (90) comprend un câble qui s'étend entre le corps principal et l'outil d'insertion quand la structure de pontage connecte le corps principal à l'outil de pontage et
l'extension du câble entre le corps principal et l'outil d'insertion est éliminée quand la structure de pontage est déconnectée.

17. Cathéter à demeure selon la revendication 1, comprenant en outre :
un dispositif de dégagement (120) positionné dans le passage intérieur et comprenant un élément de contact (122) mobile dans le passage intérieur au-delà de ladite au moins une ouverture d'entrée, pour entrer en contact et désintégrer et faciliter le mouvement des caillots sanguins et des obstructions dans ladite ouverture d'entrée et le passage intérieur avec le flux d'urine à travers le passage intérieur.

18. Cathéter à demeure selon la revendication 17, dans lequel :
l'élément de contact (122) est normalement positionné dans le passage intérieur dans une position arrimée, entre chaque ouverture d'entrée et une extrémité distale du passage intérieur et
l'élément de contact (122) est mobile depuis la position arrimée vers une position proximale de chaque ouverture d'entrée dans le passage intérieur à travers le corps principal.

19. Cathéter à demeure selon la revendication 17, comprenant en outre :
un dispositif d'activation (128) connecté à l'élément de contact (122) et s'étendant à travers le canal urinaire vers une position située en dehors d'une ouverture extérieure du canal urinaire ; et dans lequel :
l'élément de contact (122) est sélectivement déplacé par manipulation du dispositif d'activation (128) vers la position située en dehors de l'ouverture extérieure du canal urinaire.

20. Cathéter selon la revendication 19, comprenant en outre, en combinaison :
un outil d'insertion (22) pour insérer le cathéter dans les voies urinaires vers la position d'utilisation, l'outil d'insertion ayant une extrémité distale à connecter à l'extrémité proximale du corps principal et une extrémité proximale qui s'étend vers une position extérieure de l'ouverture extérieure du canal urinaire, l'outil d'insertion ayant un canal intérieur s'étendant de son extrémité distale à son extrémité proximale, le canal intérieur communiquant avec le passage intérieur dans le corps principal quand l'extrémité distale de l'outil d'insertion est reliée à l'extrémité proximale du corps principal et dans lequel
le dispositif d'activation (128) s'étend à travers le canal intérieur de l'outil d'insertion quand l'outil d'insertion est connecté au corps principal et l'outil d'insertion s'étend à travers l'ouverture centrale de la section à bobine quand l'outil d'insertion est connecté au cathéter à demeure.

21. Cathéter selon la revendication 20, dans lequel :
l'élément de contact est retenu dans le passage intérieur pour enlèvement et transport à travers le passage intérieur et hors du corps principal par manipulation du dispositif d'activation dans la position extérieure à l'ouverture externe du canal urinaire.

22. Cathéter selon la revendication 17, comprenant en outre :
un élément de retour (126) connecté entre l'élément de contact et le corps principal, afin d'incliner l'élément de contact dans une position initiale à partir de laquelle l'élément de contact commence le mouvement et
l'élément de retour (126) déplaçant l'élément de contact de nouveau dans la position initiale, après que l'élément de contact a été déplacé au-delà de l'ouverture d'entrée et le long du passage intérieur.

23. Cathéter à demeure selon la revendication 1, dans lequel :
l'élément de retour (126) comprend un ressort.

24. Cathéter selon la revendication 19, dans lequel :
le dispositif d'activation (128) comprend une attache connectée à l'élément de contact (122) pour déplacer l'élément de contact au-delà de l'ouverture d'entrée et le long du passage intérieur, lors de la traction de l'attache dans la position à l'extérieur de l'ouverture extérieure du canal urinaire.

25. Cathéter selon la revendication 17, dans lequel :
l'élément de contact (122) est retenu dans le passage intérieur pour enlèvement et transport à travers le passage intérieur et hors du corps principal en tirant l'attache après déplacement de l'élément de contact au-delà de l'ouverture d'entrée.

26. Cathéter selon la revendication 17, dans lequel :
l'élément de contact comprend une structure en brosse.

27. Cathéter selon la revendication 26, dans lequel :
la structure à brosse comprend une pluralité de crins.

28. Cathéter selon la revendication 27, dans lequel :
la structure à brosses est maintenue dans le passage intérieur par frottement entre les crins et le passage intérieur jusqu'au déplacement de l'élément de contact.

29. Cathéter selon la revendication 1, comprenant en outre :
un revêtement à faible frottement s'étendant sur l'extrémité distale du corps principal, dans chaque ouverture d'entrée, et le long du passage intérieur.
